(19) **Europäisches Patentamt / European Patent Office / Office européen des brevets**

(11) **EP 3 565 656 B1**

(12) **FASCICULE DE BREVET EUROPEEN**

(45) Date de publication et mention de la délivrance du brevet:
**13.08.2025   Bulletin 2025/33**

(21) Numéro de dépôt: **18700791.9**

(22) Date de dépôt: **05.01.2018**

(51) Classification Internationale des Brevets (IPC):
**B01D 61/12** *(2006.01)*     **C07H 3/02** *(2006.01)*
**C12P 19/02** *(2006.01)*     **A23L 27/30** *(2016.01)*

(52) Classification Coopérative des Brevets (CPC):
(C-Sets disponibles)
**C07H 1/00; A23L 27/33; B01D 61/027;
B01D 61/04; C07H 3/02;** B01D 2311/04;
B01D 2311/2643; B01D 2311/2676;
B01D 2311/2697                                    (Cont.)

(86) Numéro de dépôt international:
**PCT/FR2018/050026**

(87) Numéro de publication internationale:
**WO 2018/127668 (12.07.2018 Gazette 2018/28)**

(54) **PROCÉDÉ DE FABRICATION DE CRISTAUX DE D-ALLULOSE**

VERFAHREN ZUR HERSTELLUNG VON D-ALLULOSE-KRISTALLEN

METHOD FOR PRODUCING D-ALLULOSE CRYSTALS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **05.01.2017  FR 1750103**

(43) Date de publication de la demande:
**13.11.2019   Bulletin 2019/46**

(73) Titulaire: **Roquette Frères**
**62136 Lestrem (FR)**

(72) Inventeurs:
  • **BOIT, Baptiste**
    **59253 La Gorgue (FR)**
  • **LACROIX, Geoffrey**
    **59160 Lille (FR)**
  • **ROSSI, Laurent**
    **62000 Arras (FR)**

(74) Mandataire: **Plasseraud IP**
**104 Rue de Richelieu**
**CS92104**
**75080 Paris Cedex 02 (FR)**

(56) Documents cités:
EP-A1- 1 860 195          WO-A1-2016/064087
WO-A2-2011/119004     CN-A- 103 333 935
CN-A- 104 447 888         CN-B- 103 059 071
FR-A1- 3 016 628

• TAKESHITA K ET AL: "Mass production of D-psicose from D-fructose by a continuous bioreactor system using immobilized D-tagatose 3-epimerase", JOURNAL OF BIOSCIENCE AND BIOENGINEERING, ELSEVIER, AMSTERDAM, NL, vol. 90, no. 4, 1 January 2000 (2000-01-01), pages 453 - 455, XP003003993, ISSN: 1389-1723

• FAKHREDDIN SALEHI: "Current and future applications for nanofiltration technology in the food processing", FOOD AND BIOPRODUCTS PROCESSING, vol. 92, no. 2, 1 April 2014 (2014-04-01), GB, pages 161 - 177, XP055369772, ISSN: 0960-3085, DOI: 10.1016/j.fbp.2013.09.005

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)

C-Sets
B01D 2311/04, B01D 2311/2676

(52) Classification Coopérative des Brevets (CPC):
(Cont.)

C-Sets
B01D 2311/04, B01D 2311/2676

## Description

### Domaine de l'invention

L'invention porte sur un nouveau procédé de fabrication de cristaux de D-allulose qui permet de travailler en continu et obtenir un haut rendement. L'invention a également pour objet de nouveaux cristaux de D-allulose. Un autre objet de l'invention porte sur l'utilisation d'une unité de nanofiltration dans un circuit de production de cristaux D-allulose pour améliorer le rendement global en cristaux de D-allulose.

L'invention est définie par les revendications annexées.

### Art antérieur

Le D-allulose (ou D-psicose) est un sucre rare présentant un pouvoir sucrant égal à 70% de celui du saccharose. Contrairement à ce dernier, le D-allulose ne provoque pas de prise de poids car il n'est pas métabolisé par les humains. Il présente une très faible valeur calorique (0,2 kcal par gramme) et il empêche ainsi la prise de masse graisseuse. De plus, des études ont montré que le D-allulose est non cariogène, voire anti-cariogène. C'est ainsi que ces propriétés ont généré récemment un intérêt très important des industries alimentaires et pharmaceutiques.

Même s'il est possible d'obtenir du D-allulose par voie chimique, par exemple en faisant réagir une solution aqueuse de glucose en milieu acide en présence d'un catalyseur de type molybdate d'ammonium, le D-allulose est généralement obtenu par voie enzymatique, en faisant réagir une solution aqueuse de D-fructose avec une D-psicose epimerase telle que décrite par exemple dans la demande WO2015/032761 A1 au nom de la Demanderesse. Dans les deux cas, la réaction n'est pas totale. Par exemple, la quantité de D-fructose transformée en D-allulose après épimérisation est généralement inférieure à 30%.

Ainsi, à l'issue de la réaction d'épimérisation, il est nécessaire de réaliser une étape de séparation du D-allulose, pour augmenter la richesse de la composition de D-allulose résultante. Pour réaliser cette séparation, on effectue très généralement une chromatographie de la composition issue de la réaction d'épimérisation, par exemple par chromatographie continue de type lit mobile simulé.

Le document JP2001354690 A décrit un procédé de fabrication d'un sirop de D-allulose partant d'un mélange de fructose et de D-allulose, ledit procédé comprenant une étape de séparation consistant en une étape de chromatographie continue utilisant une séquence particulière de prélèvements des différents produits du mélange. Une fraction riche en D-allulose (dont la richesse en D-allulose peut atteindre 98%) et une fraction riche en fructose sont récupérées. Le rendement de récupération dans la fraction riche en D-allulose est de 96%.

A l'issue des étapes de séparation citées ci-dessus, des compositions liquides riches en D-allulose sont obtenues. C'est ainsi que ces compositions liquides, généralement appelés sirops, sont utilisées pour la fabrication de produits alimentaires ou pharmaceutiques.

A titre d'exemple, la demande WO 2015/094342 également au nom de la Demanderesse décrit la fabrication de produits alimentaires solides comprenant un sirop de D-allulose, comprenant de 50 à 98% de D-allulose et une protéine native. C'est principalement sous cette forme de sirops que différentes sociétés ont annoncé la commercialisation de D-allulose à ce jour.

Le D-allulose est également commercialisé sous forme de poudres. Toutefois, comme expliqué ci-dessous, leurs fabrications peuvent être assez complexes.

Il est possible de réaliser des poudres en utilisant par exemple des techniques d'atomisation. Toutefois, les poudres atomisées, dont la composition dépend de la matière première atomisée, comprennent généralement de grandes quantités impuretés. Par ailleurs, ces poudres atomisées sont faiblement cristallines ; elles sont très hygroscopiques et cela provoque ainsi des problèmes de tenue à l'eau. Cela crée en outre des phénomènes de mottage généralement plus importants.

Du fait de ces difficultés, l'atomisation de D-allulose a été peu décrite dans la littérature. A titre d'exemple, on peut citer le document EP1860195 qui décrit l'atomisation d'un mélange comprenant du D-allulose et du D-allose. En ce qui concerne l'énantiomère du D-allulose, le L-allulose, on peut citer le document JP 4761424 qui décrit l'atomisation d'une masse cuite de L-allulose.

Il est également possible de réaliser des poudres par des techniques de granulation, tel que décrit par exemple dans la demande WO2016/012853 au nom de la Demanderesse.

Une autre forme de poudres concerne des cristaux obtenus par cristallisation d'une solution mère de D-allulose.

Un procédé de fabrication de cristaux de D-allulose a été décrit dans le document CN 104447888 A. Ce document décrit plus particulièrement dans les exemples un procédé comprenant une étape de fabrication d'une solution de D-allulose à partir de glucose en utilisant un catalyseur molybdate puis une étape de décoloration de cette solution en utilisant du charbon actif puis une filtration pour éliminer le charbon actif, une étape de déionisation par électrodialyse, une étape de séparation par chromatographie continue pour former une solution de D-allulose présentant une pureté allant de 70 à 90%, une étape de concentration de ladite solution pour obtenir une solution concentrée de D-allulose, suivie d'une étape de cristallisation dans l'éthanol pour obtenir un produit cristallin de D-allulose présentant une pureté limitée, tout au plus de 99%. Aucun rendement de cristallisation n'est indiqué dans ce document.

Un autre procédé de cristallisation a également été décrit dans le document WO 2011/119004 au nom de CJ Cheiljedang dans lequel le rendement de cristallisation est de l'ordre de 50%. Ce procédé de fabrication de cristaux de D-allulose comprend une étape de fourniture d'une solution de D-allulose, une étape de purification de cette solution, une étape de concentration de la solution de D-allulose pour fournir une solution mère et une étape de cristallisation de cette solution mère, dans lequel l'étape de cristallisation est réalisée en maintenant la solution mère dans sa zone métastable. L'étape de purification décrite dans ce document est une étape de séparation par chromatographie, qui permet de séparer le D-allulose du fructose. Ce document reconnaît toutefois que la fabrication de poudres sous forme de cristaux est difficile à mettre en œuvre :

- Tout d'abord, cette cristallisation est difficile à contrôler comme il est indiqué au paragraphe [36], dans lequel il est précisé que la cristallisation doit être conduite avec soin, par une observation continue des cristaux, en mesurant également la concentration du surnageant, ceci afin de régler la température dans le cristallisoir. Toutefois, ce document est silencieux sur les raisons pour lesquelles il est difficile de réaliser cette cristallisation.

- De plus, les cristaux obtenus présentent une taille inférieure à 200 $\mu m$ (voir paragraphe [89]) et sont donc, du fait de leur faible taille, difficilement séparables des eaux mères de cristallisation lors de la centrifugation. Les cristaux récupérés sont également difficilement manipulables lors d'une utilisation ultérieure.

Le document WO 2016/064087, également au nom de CJ Cheiljedang, décrit également à l'exemple 3 un procédé de fabrication de cristaux de D-allulose dans lequel une solution mère est introduite dans un cristallisoir à quatre moments distincts. Entre chaque introduction, on réalise quatre cycles de chauffe et de refroidissement, ce qui conduit à un procédé qui dure plus de 80 heures. En comparaison avec le procédé décrit dans le document WO 2011/119004, le rendement de cristallisation n'est pas amélioré (il est de 52,8%). Les cristaux obtenus ont une taille moyenne plus importante (mean aperture égale à 374 $\mu m$). Toutefois, les cristaux obtenus présentent des impuretés. Par ailleurs, les cristaux qui sont commercialisés par la société CJ Cheiljedang ont une forme d'aiguilles, ce qui conduit à un écoulement qui n'est pas totalement satisfaisant.

Il apparaît de ce qui précède que de nombreux problèmes subsistent encore dans la fabrication de cristaux de D-allulose.

Tout d'abord, le rendement global en cristaux de D-allulose est excessivement faible. Par « rendement global en cristaux de D-allulose », on entend le rapport, exprimé en masse sèche, de la masse de cristaux de D-allulose obtenue sur la masse de D-fructose introduite. Ce faible rendement, de l'ordre de 15% par rapport au D-fructose, est essentiellement lié aux rendements des étapes d'épimérisation (inférieur à 30%) et de cristallisation (généralement de l'ordre de 50%).

Afin d'améliorer le rendement des procédés, il est donc impératif de réaliser « des étapes de recyclage ». Par « étape de recyclage » dans un procédé, on entend la réutilisation dans une étape antérieure du procédé d'une fraction de produit obtenue lors d'une étape de séparation. Par « étape de séparation » dans la présente Demande, on entend toute étape permettant de séparer une composition comprenant un produit A et un produit B en au moins une première fraction plus riche en produit A et une deuxième fraction plus riche en produit B. La fraction peut être sous toute forme, par exemple sous forme solide, sous forme liquide, voire sous forme de suspension solide dans un liquide. Une étape de séparation peut être de tout type, par exemple une étape de chromatographie, dans laquelle la composition liquide est séparée en au moins deux fractions liquides, ou encore une étape de cristallisation où une composition liquide est séparée en une fraction solide et une fraction liquide.

Des étapes de recyclages de fractions riches en fructose ont déjà été décrites en vue d'améliorer le rendement du procédé de fabrication de sirops de D-allulose. Notamment, le document JP2001354690 A précédemment évoqué décrit le recyclage de la fraction riche en fructose obtenue lors de l'étape de chromatographie pour la soumettre à l'étape d'épimérisation. Ceci permet d'améliorer le rendement de D-allulose sous forme de composition liquide.

C'est en cherchant à appliquer les enseignements de ce document dans un procédé de fabrication de cristaux de D-allulose (tel que par exemple celui décrit dans le document WO 2011/119004) que la Demanderesse a pu constater que ceux-ci n'étaient pas simplement transposables.

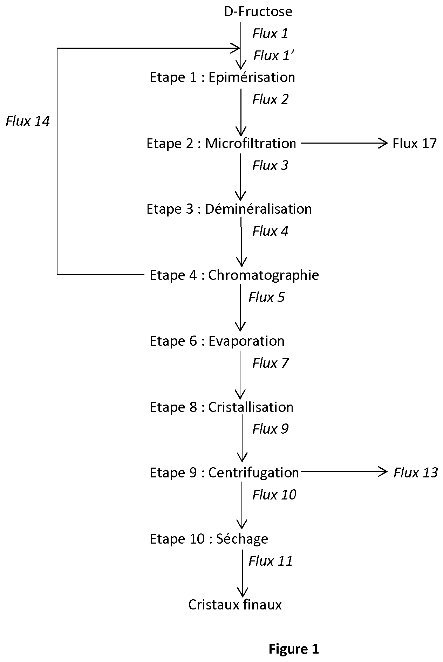

En effet, lorsque l'on effectue le recyclage de la fraction riche en fructose (voir Figure 1), la Demanderesse a pu constater que la solution mère obtenue devient de plus en plus difficile à cristalliser. Au bout d'un certain temps, cette cristallisation devient même impossible comme démontré dans la partie Exemples.

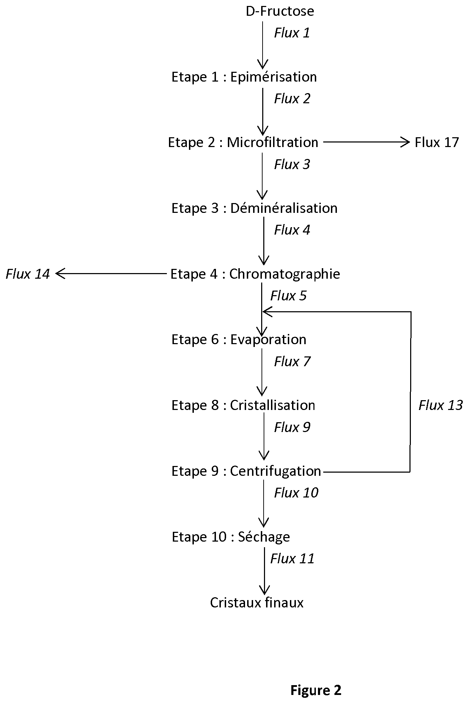

Par ailleurs, toujours dans le but d'améliorer le rendement global en cristaux de D-allulose du procédé, la Demanderesse a également tenté d'effectuer un recyclage des eaux mères de cristallisation (c'est-à-dire de la solution riche en D-allulose qui est obtenue après séparation des cristaux obtenus lors de l'étape de cristallisation) en les mélangeant avec une composition de D-allulose pour former, après concentration, une nouvelle solution mère (voir Figure 2). Elle a pu constater (voir partie Exemples) que ce recyclage des eaux mères provoquait les mêmes difficultés, et ceci de manière encore plus rapide que dans le cas du recyclage de la fraction riche en fructose.

Dans ces deux cas décrits précédemment, des arrêts dans la production des cristaux de D-allulose doivent donc être réalisés.

Par ailleurs, la Demanderesse a pu constater que, même sans réaliser de recyclages, en transposant les enseignements décrits ci-dessus dans un procédé industriel continu de fabrication de cristaux de D-allulose, des « instabilités » sont observées. Ces instabilités se traduisent par des masses-cuites de cristaux présentant de manière inexplicable un aspect variable au cours du procédé. Ceci est particulièrement gênant car les masses-cuites de cristaux peuvent être centrifugeables puis non centrifugeables à certains moments, et ceci sans qu'aucune prédiction a priori de son comportement puisse être réalisé. Il y a alors besoin de refondre les masses cuites obtenues si l'on souhaite les réutiliser dans le procédé et ceci rend le procédé peu pratique et moins économique.

Or, en vue d'accélérer le développement industriel du D-allulose, qui est aujourd'hui principalement commercialisé sous la forme de sirop, il est impératif à l'échelle industrielle de pouvoir réaliser un procédé continu et stable de fabrication de cristaux de D-allulose, afin de pouvoir les fournir à un prix compétitif.

Après de nombreuses recherches, la Demanderesse est parvenue à obtenir un procédé de fabrication de cristaux de D-allulose dans lequel les problèmes cités ci-dessus peuvent être résolus.

Du fait d'une stabilité de procédé améliorée, la production des cristaux ne nécessite pas de contrôle systématique comme par exemple indiqué dans le document WO 2011/119004, ce qui rend possible la réalisation d'un procédé continu de fabrication de cristaux de D-allulose.

Par ailleurs, comme le procédé de l'invention permet en outre de nombreuses étapes de recyclage, le rendement du procédé peut être grandement augmenté.

C'est en menant de nombreuses recherches que la Demanderesse a pu constater que, dans un procédé de fabrication de cristaux de D-allulose, des impuretés particulières se forment au cours des différentes étapes du procédé de préparation de la solution mère. Ces impuretés n'ont, à la meilleure connaissance de la Demanderesse, jamais été reportées dans la littérature. Elles ont pu être identifiées par la Demanderesse en utilisant une technique particulière de chromatographie phase gaz (CPG). Sans être liée à une quelconque théorie, la Demanderesse pense que ces impuretés sont des dimères de D-allulose qui se forment par condensation tout le long du procédé.

La Demanderesse a également pu montrer que ces dimères de D-allulose, contrairement à d'autres impuretés telles que le glucose ou le D-fructose, ont un effet anti-cristallisant très important.

De manière surprenante, la Demanderesse est parvenue, en réalisant un procédé de fabrication de cristaux de D-allulose dans lequel une étape de séparation particulière est réalisée, à supprimer ce problème de cristallisation difficile du D-allulose. Cette étape de séparation consiste en une étape de nanofiltration, qui permet l'élimination de ces impuretés anti-cristallisantes.

Ce nouveau procédé présente une avancée majeure pour le développement industriel de cristaux de D-allulose puisqu'il permet d'atteindre un rendement global dépassant 25% par rapport au D-fructose introduit, avantageusement dépassant 50%, voire dépassant 65%. Cela permet d'envisager la fabrication de cristaux de D-allulose à un prix compétitif ainsi que le développement commercial de tels cristaux à plus grande échelle.

Par ailleurs, la Demanderesse a constaté que la présence de ces impuretés est un facteur qui influe sur la forme des cristaux, notamment lorsque l'on cherche à réaliser des cristaux de grande taille, notamment lorsque leur taille moyenne dépasse 200 μm.

Lors de ses recherches, la Demanderesse est également parvenue à fabriquer de nouveaux cristaux de D-allulose en utilisant un procédé de cristallisation spécifique utilisant, parmi les étapes de fabrication, l'étape de nanofiltration mentionnée ci-dessus. Ces cristaux, qui sont un autre aspect de la présente invention, présentent notamment la particularité de comprendre un pourcentage massique en dimère de D-allulose très faible. Les cristaux obtenus peuvent présenter en outre une forme différente et un écoulement amélioré. Ces propriétés de forme et d'écoulement sont directement liées au fait que les dimères sont présents dans les cristaux dans de très faibles quantités. Au contraire, lorsque l'on souhaite réaliser des cristaux de grande taille, la présence importante de dimères D-allulose dans la solution mère lors de la cristallisation conduit à des cristaux de D-allulose comprenant des quantités importantes de ces dimères ; par ailleurs, on observe l'allongement de ces cristaux pour former des aiguilles. Or, ces cristaux en aiguille peuvent présenter un écoulement moindre que les cristaux de l'invention.

### Résumé de l'invention

L'invention porte ainsi sur un procédé de fabrication de cristaux de D-allulose tel que défini à la revendication 1 comprenant :

- une étape de fourniture d'une composition riche en D-allulose,
- une étape de nanofiltration de ladite composition riche en D-allulose pour fournir un rétentat et un perméat, l'étape de nanofiltration étant réalisée avec une membrane ayant un seuil de coupure inférieur à 300 Da, de préférence allant de 150 à 250 Da;

- une étape de récupération du perméat de nanofiltration ;
- une étape de concentration de ce perméat pour fournir la solution mère de D-allulose ;
- une étape de cristallisation de la solution mère pour former des cristaux de D-allulose et des eaux mères.

Comme évoqué ci-dessus, la Demanderesse a pu constater que, systématiquement, dans un procédé de fabrication de cristaux de D-allulose, des impuretés particulières se forment au cours du procédé. Celles-ci n'ont, à la meilleure connaissance de la Demanderesse, jamais été reportées dans la littérature. Ceci s'explique par le fait que, par la technique de chromatographie liquide haute performance (HPLC) classiquement utilisée pour mesurer la pureté du D-allulose, ces impuretés ne sont pas détectées sur les chromatogrammes (voir Figures 7 et 8). C'est en utilisant une technique de chromatographie en phase gaz que la Demanderesse a pu détecter leur présence (voir Figures 9 et 10).

Ces impuretés ont pu être identifiées par la Demanderesse comme étant des dimères de D-allulose. La Demanderesse a également pu montrer que ces dimères, contrairement à d'autres impuretés telles que le glucose ou le D-fructose, ont un effet anti-cristallisant très important. Or, comme ces dimères de D-allulose se forment lors du procédé, leur présence peut limiter le rendement d'un procédé continu de fabrication de cristaux de D-allulose en empêchant de manière pure et simple cette cristallisation, en formant des masses-cuites non centrigugeables, si les quantités en ces dimères sont trop importantes. Par ailleurs, dans un procédé industriel et continu de fabrication de cristaux de D-allulose où de nombreuses étapes successives sont réalisées, la quantité de ces impuretés peut varier au cours du temps. Ceci rend le procédé instable au cours du temps, les masses-cuites étant parfois centrifugeables, parfois non centrifugeables.

Il est du mérite de la Demanderesse d'avoir identifié ces impuretés spécifiques et d'être parvenu à les éliminer, en réalisant une étape de séparation consistant à séparer au moins en partie ces dimères de D-allulose par de nanofiltration.

A l'aide du procédé de l'invention qui rend l'étape de cristallisation très stable, il est alors possible de conduire le procédé de manière continue mais également d'augmenter de manière drastique le rendement global en cristaux de D-allulose. Ceci est permis par le fait de pouvoir fournir une solution mère de D-allulose présentant une très faible quantité de dimères de D-allulose, ceci grâce à l'étape de nanofiltration susmentionnée.

Un autre mérite de la Demanderesse est d'être parvenue à fournir de nouveaux cristaux de D-allulose en utilisant cette solution mère, dans un procédé particulier de cristallisation qui limite également la formation in-situ desdits dimères. Il en résulte des cristaux dont le pourcentage massique de dimères de D-allulose est plus faible que ceux de l'art antérieur. Un autre objet de l'invention porte ainsi sur des cristaux de D-allulose tels que définis à la revendication 13 comprenant une teneur massique en dimère de D-allulose, déterminée par chromatographie en phase gaz (CPG), inférieure à 0.5%, et présentant une taille moyenne en volume D4,3 supérieure à 200 $\mu$m, avantageusement allant de 210 à 800 $\mu$m, préférentiellement de 220 à 350 $\mu$m, et pour une taille de particules en volume D4,3 donnée et choisie dans la gamme allant de 200 à 400 $\mu$m, un rapport Feret min/Feret max supérieur à 0,60, avantageusement allant de 0,62 à 0,90, par exemple de 0,63 à 0,80.

En ce qui concerne les cristaux décrits dans le document WO2011119004 A2, comme ils présentent une taille très fine, ils sont difficiles à séparer des eaux mères de cristallisation. Ces eaux mères comprenant de grandes quantités de dimères de D-allulose et la teneur en dimères dans les cristaux récupérés après séparation est donc importante, bien supérieure à celle des cristaux de l'invention. Par ailleurs, l'exemple comparatif 3 de la présente Demande (voir parties exemples) démontre que même en améliorant encore le procédé décrit dans ce document, la Demanderesse n'est pas parvenue à obtenir les cristaux de l'invention.

Quant aux cristaux décrits dans le document WO 2016/064087, ils présentent des impuretés, qui peuvent être des dimères de D-allulose dans des quantités supérieures aux cristaux de l'invention. Sans être lié à une quelconque théorie, la présence de ces dimères peut être expliquée par le fait que la solution de D-allulose est maintenue à la chaleur avant d'être introduite dans le cristallisoir, *i.e.* pendant une durée atteignant 60 heures. De plus, ce document est totalement silencieux sur la manière de préparer la solution mère, notamment en ce qui concerne les conditions de l'étape de concentration. Or, comme il apparaît dans la suite de la description, le choix des conditions des étapes de cristallisation et de concentration ont un impact important sur la quantité de dimères de D-allulose formés. Ainsi, le choix de ces conditions permet de limiter les quantités de dimères formés lors du procédé et d'ainsi diminuer les teneurs de dimères de D-allulose dans les cristaux de D-allulose obtenus. Comme le choix des conditions des étapes de préparation de la solution mère ont un impact capital sur la quantité de dimères de D-allulose dans celle-ci, ce document ne décrit ni la solution mère utile à la préparation des cristaux de l'invention, ni les cristaux eux-mêmes.

Le document EP1860195 décrit des compositions cristallines complexes de type tubulaires de D-allose et D-allulose où le D-allulose est minoritaire et non des cristaux de D-allulose.

Pour les compositions cristallines obtenues par cristallisation dans l'éthanol décrites dans le document CN 104447888 A, ce document est silencieux sur de nombreuses conditions de procédé de fabrication des compositions cristallines et notamment sur l'étape de concentration de la solution mère. Il est à ce titre impossible de reproduire ces essais de fabrication des compositions cristallines. Toutefois, il est utile de noter que la cristallisation doit être faite spécifiquement faite dans un solvant organique tel que l'éthanol. Or, il est connu qu'une cristallisation dans ce type de solvant est utilisée uniquement si nécessaire. En effet, l'eau est généralement préferée pour des raisons évidentes de coûts et environnementales. Un solvant organique sélectionné (l'éthanol dans le cas du D-allulose), bien que plus coûteux et imposant des difficultés pour son retraitement, présente l'avantage de faciliter la cristallisation, rendant possible une cristallisation qui serait impossible dans l'eau. Ceci confirme donc que la solution mère de D-allulose comprend de grandes quantités d'impuretés de type dimères de D-allulose et ceci peut s'expliquer par le fait qu'aucune précaution particulière pour les différentes étapes de préparation de la solution mère ne semble avoir été réalisée (notamment aucune condition n'est indiquée pour l'étape de concentration). Par ailleurs, en termes de purification, une cristallisation dans l'éthanol peut être moins efficace pour obtenir des cristaux de pureté élevée de D-allulose qu'une cristallisation dans de l'eau (quand celle-ci est possible) car ces dimères de nature chimique proche du D-allulose sont également peu solubles dans l'éthanol et précipitent également. Enfin, d'autres impuretés également nombreuses se retrouvent dans les compositions cristallines comme le démontrent les faibles puretés obtenues dans ce document. Des compositions cristallines obtenues par cristallisation dans l'éthanol sont également décrites dans la publication de Takeshita et al. (Mass production of D-psicose from D-fructose by a continuous bioreactor system using immobilized D-tagatose 3-epimerase, Journal of bioscience and bioengineering, Vol.90 n°4, janvier 2000, pages 453-455). Ce document se concentre sur la première étape de préparation du D-allulose et non sur la fabrication des cristaux de D-allulose qui sont là-aussi très peu détaillées. Comme le choix des conditions des étapes de préparation de la solution mère ont un impact capital sur la quantité de dimères de D-allulose dans celle-ci, ce document ne décrit ni la solution mère utile à la préparation des cristaux de l'invention, ni les cristaux eux-mêmes. Par ailleurs, dans ce document, il est précisé que cette cristallisation est réalisée dans l'éthanol pour faciliter la solidification du D-allulose, ce qui démontre la quantité importante en impuretés non cristallisantes et confirme l'impossibilité de cristalliser la solution mère de D-allulose de ce document dans de l'eau. D'ailleurs, la cristallisation en question dans ce document est en réalité qu'une « précipitation » non contrôlée par ajout d'éthanol, menant inévitablement à des compositions solides cristallines impures.

D'autres composition cristallines sont également évoquées dans le document CN 103333935 A mais sans aucun détail de leur préparation puisqu'aucune condition de préparation de la solution-mère ni de cristallisation (pas même de solvant) ne sont indiquées. Par ailleurs ce document ne comprend pas d'essai de préparation à proprement dit. Comme le choix des conditions des étapes de préparation de la solution mère ont un impact capital sur la quantité de dimères de D-allulose dans celle-ci, ce document ne décrit ni la solution mère utile à la préparation des cristaux de l'invention, ni les cristaux eux-mêmes. Par ailleurs, les faibles puretés des compositions cristallines obtenues (98%) dans ce document semblent indiquer que les compositions cristallines sont très impures.

Un autre objet de l'invention porte également sur l'utilisation telle que définie à la revendication 16 d'une unité de nanofiltration dans un circuit de production de cristaux de D-allulose pour améliorer le rendement global en cristaux de D-allulose, la membrane utilisée pour la nanofiltration ayant un seuil de coupure inférieur à 300 Da, de préférence allant de 150 à 250 Da.

## Brève description des Figures

Figure 1 : La Figure 1 représente schématiquement un circuit de production de cristaux de D-allulose dans lequel le raffinat de chromatographie riche en D-fructose est recyclé pour être mélangé avec le fructose en tête de la réaction d'épimérisation.

Figure 2 : La Figure 2 représente schématiquement un circuit de production de cristaux de D-allulose dans lequel les eaux mères de cristallisation sont recyclées pour être mélangées avec la composition de D-fructose/D-allulose issue de la réaction d'épimérisation.

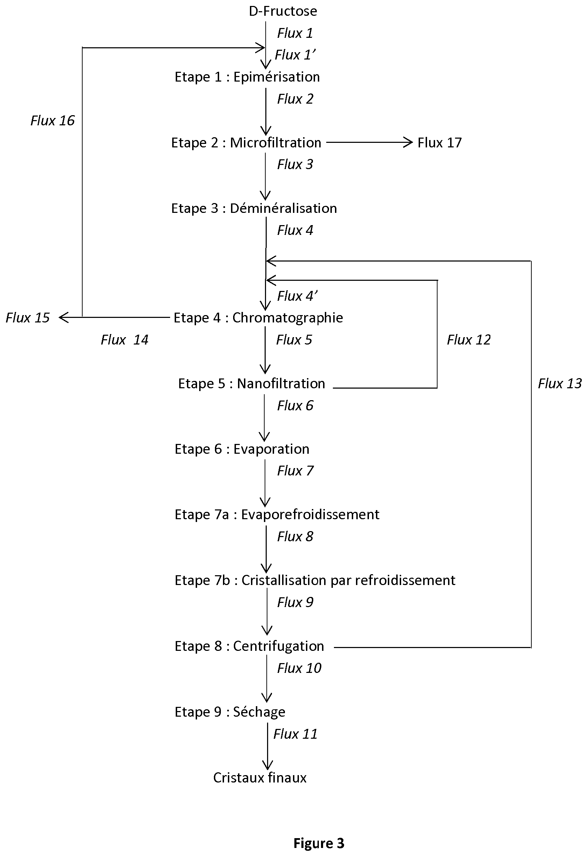

Figure 3 : La Figure 3 représente schématiquement un circuit de production de cristaux de D-allulose comprenant une unité de nanofiltration utile au procédé de l'invention.

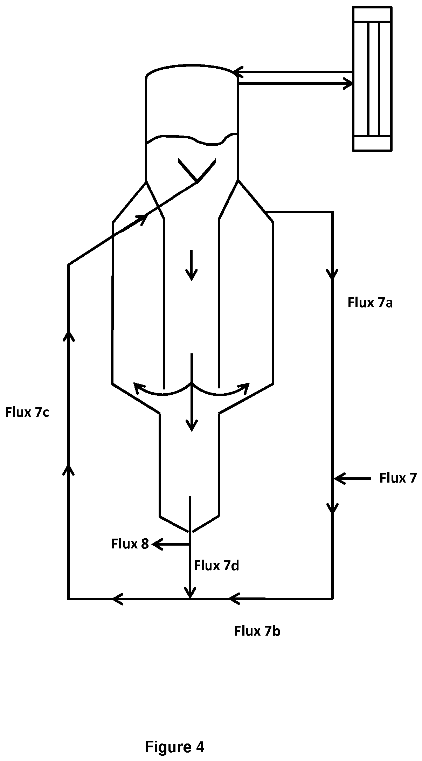

Figure 4 : La Figure 4 représente un exemple de cristallisoir-évaporateur adiabatique sous vide utile à une variante du procédé de l'invention.

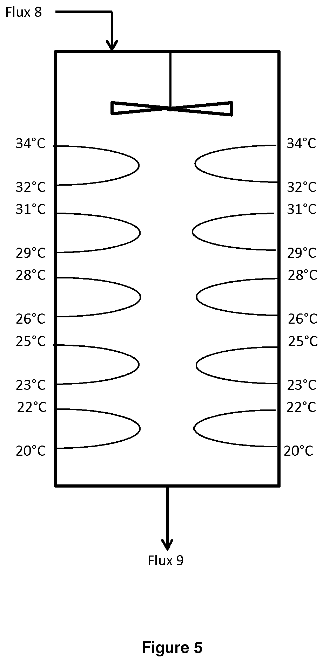

Figure 5 : La Figure 5 représente un exemple de cristallisoir vertical utile à une variante du procédé de l'invention.

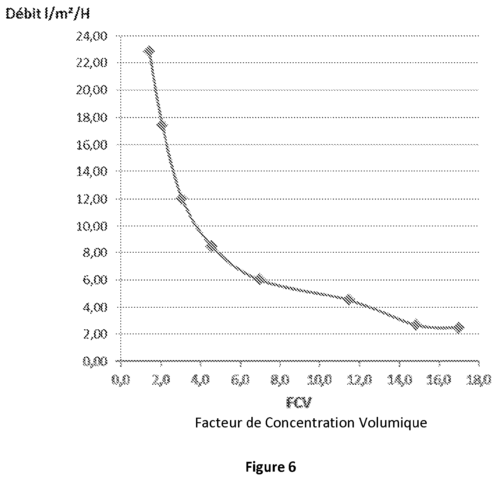

Figure 6 : La Figure 6 représente la courbe de perméation relative à l'étape de nanofiltration, c'est-à-dire le débit en fonction du facteur de concentration volumique.

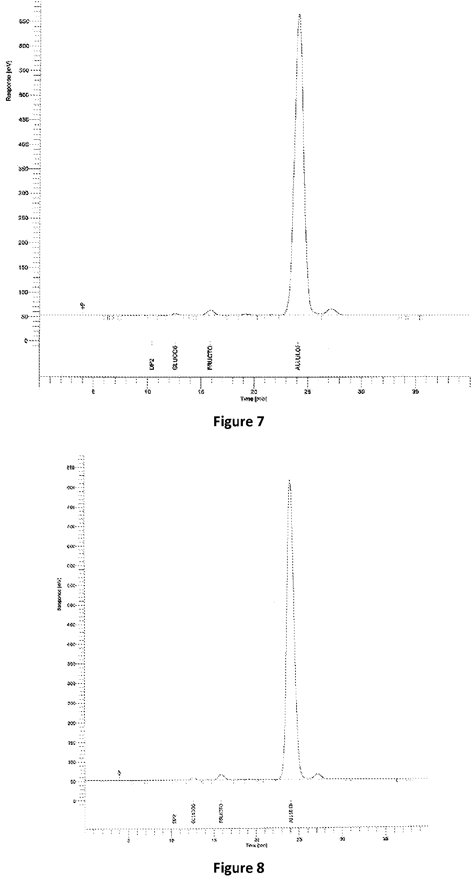

Figure 7 : La Figure 7 représente un chromatogramme HPLC d'une composition riche en D-allulose prélevée dans le procédé de l'invention (voir Figure 3), c'est-à-dire avant nanofiltration.

Figure 8 : La Figure 8 représente un chromatogramme HPLC d'un perméat prélevé dans le procédé de l'invention (voir Figure 3), c'est-à-dire après nanofiltration.

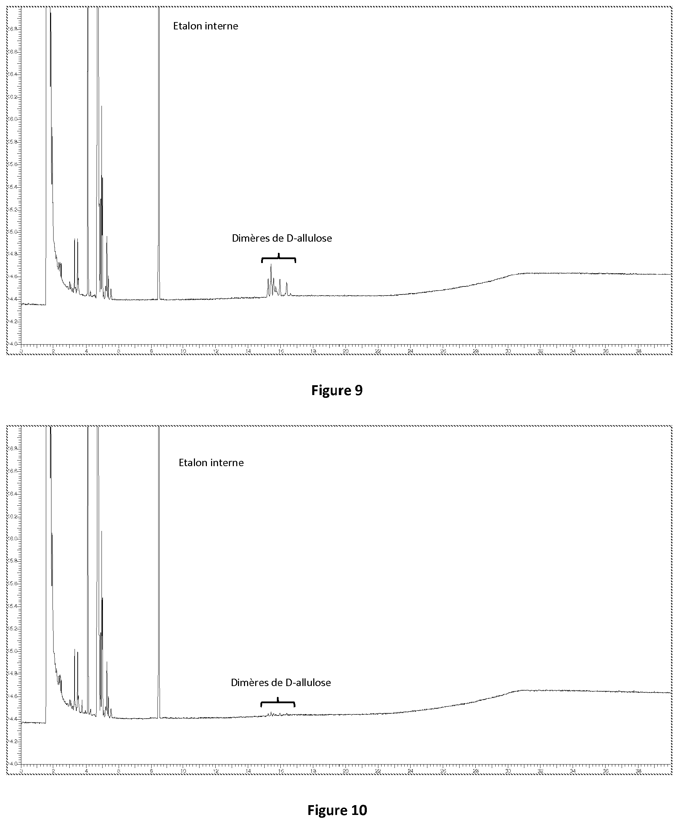

Figure 9 : La Figure 9 représente un chromatogramme CPG, dans la zone caractéristique des dimères, d'une composition riche en D-allulose prélevée dans le procédé de l'invention (voir Figure 3), c'est-à-dire avant nanofiltration.

Figure 10 : La Figure 10 représente un chromatogramme CPG, dans la zone caractéristique des dimères, d'un perméat prélevé dans le procédé de l'invention (voir Figure 3), c'est-à-dire après nanofiltration.

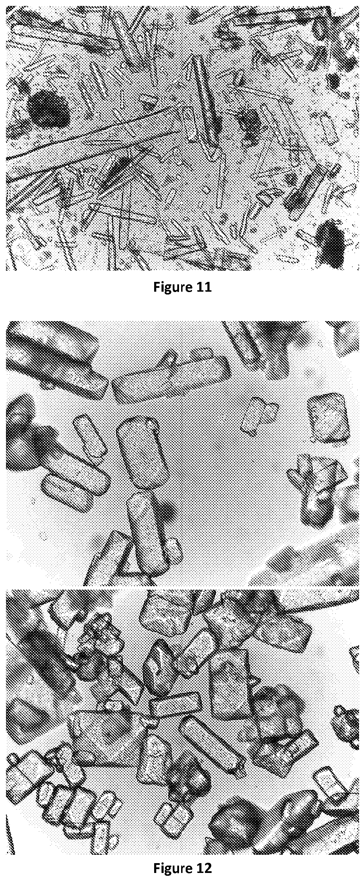

Figure 11 : La Figure 11 représente un cliché obtenu par microscopie optique de cristaux de D-allulose comparatifs.

Figure 12 : La Figure 12 représente deux clichés obtenus par microscopie optique de cristaux de D-allulose selon l'invention.

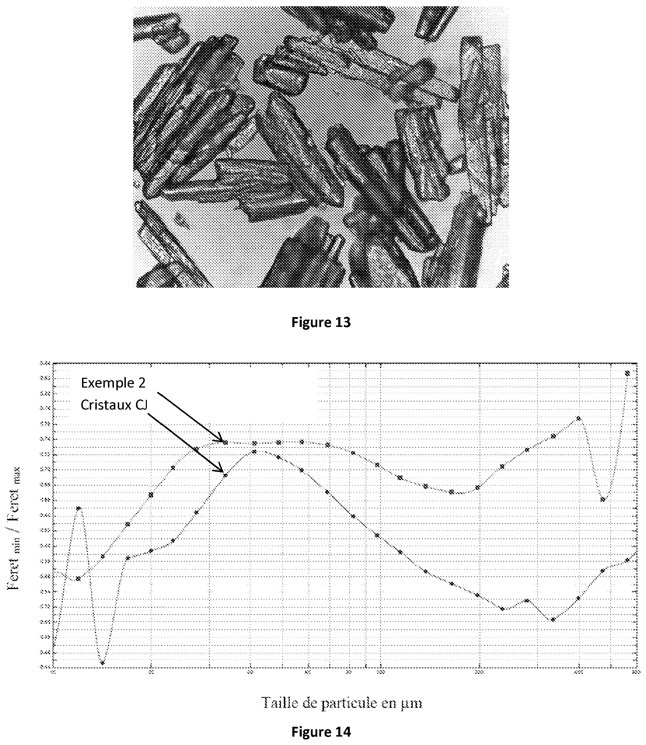

Figure 13 : La Figure 13 représente un cliché obtenu par microscopie optique de cristaux de D-allulose fabriqués et commercialisés par la société CJ CheilJedang Food Ingredient.

Figure 14 : La Figure 14 représente le rapport des diamètres Feret min/Feret max en fonction de la tailles de particules en volume D4,3 pour deux types de cristaux de D-allulose.

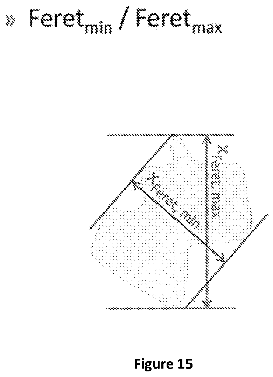

Figure 15 : La Figure 15 représente les diamètres Feret min et Feret max d'une particule modèle.

## Description détaillée de l'invention

Un procédé de fabrication de cristaux de D-allulose comprend classiquement :

- une étape de concentration d'une composition riche en D-allulose pour fournir la solution mère à cristalliser ;
- une étape de cristallisation de la solution mère pour former des cristaux de D-allulose et des eaux mères ;
- une étape de séparation des eaux mères et des cristaux de D-allulose.

Le procédé de l'invention présente la particularité de comprendre une étape de nanofiltration.
Cette étape de nanofiltration permet de limiter la quantité de dimères de D-allulose dans la solution mère fournie dans le procédé de l'invention. Cette étape de nanofiltration a lieu dans une étape antérieure à l'étape de concentration de la composition riche en D-allulose. Cette étape permet donc la fourniture d'une solution mère de D-allulose dont la teneur en dimères de D-allulose est plus faible que celle obtenue à partir d'un même procédé n'utilisant pas cette étape de nanofiltration.
Dans l'étape de nanofiltration, qui est essentielle au procédé de l'invention, deux fractions sont formées lorsque l'on soumet une composition de D-allulose à nanofiltrer :

- un perméat, qui est appauvri en dimères de D-allulose ;
- ainsi qu'un rétentat, qui est enrichi en dimères de D-allulose.

Dans la Figure 3 qui représente un circuit de production de cristaux utile au procédé de l'invention, le Flux 6 représente le perméat et le Flux 12 représente le rétentat. Pour des raisons illustrative mais non limitative, les Flux indiqués dans la suite de la description se réfèrent aux flux du circuit de production de cette Figure 3.
Le perméat de nanofiltration est un intermédiaire permettant la fabrication de cette solution mère.
Les termes « appauvri en dimères de D-allulose» et « enrichi en dimères de D-allulose » sont évidemment relatifs par rapport à la teneur en oligomères de D-allulose de la composition à nanofiltrer. Par « dimère de D-allulose », on entend un composé comprenant un D-allulose condensé avec au moins un second monosaccharide identique ou différent. Ces dimères sont par exemple des dimères de type D-allulose-D-allulose.
Ces dimères ont pu être détectés par CPG et n'ont pas pu être détectés lors de l'analyse HPLC, comme démontré dans la partie exemples. Il en découle que les quantités massiques des différents constituants, exprimées en masse sèche, sont dans la présente demande systématiquement déterminées par CPG. Pour déterminer les quantités de chacune des espèces dans la composition, l'échantillon subit généralement une étape de traitement afin de transformer les différentes espèces présentes en dérivés méthoximés triméthylsilylés. Les quantités massiques de chacune des espèces sont exprimées dans la présente Demande, sauf mention contraire, par rapport à la masse sèche totale.

Les quantités de glucose, de fructose et d'allulose peuvent être déterminées dans un chromatographe en phase gazeuse équipé d'un injecteur chauffé à 300°C, d'un détecteur à ionisation de flamme (FID) chauffé à 300°C et équipé d'une colonne DB1 capillaire de 40 mètres, présentant un diamètre interne de 0,18 mm et une épaisseur de film de 0,4 $\mu$m, la température de la colonne étant programmée de manière suivante : de 200°C jusqu'à 260°C à raison de 3°C/min, puis de 260 °C jusqu'à 300°C à 15°C/min, maintien à 300°C pendant 5 min. Par quantité de dimères de D-allulose, on entend la différence entre la quantité totale de dimères dans un échantillon, déterminée par CPG, et la quantité des dimères connus éventuellement présents, qui sont des dimères glucose-glucose tels que le maltose et l'isomaltose. Toutefois, la quantité de ces dimères glucose-glucose est généralement très faible, voire inexistante. Par exemple, dans la solution mère utile à l'invention, la quantité massique de dimères glucose-glucose est généralement inférieure à 0,2%, souvent inférieure à 0,1%. Il en est de même des cristaux de l'invention.

La quantité éventuelle de dimères glucose-glucose peut être déterminée dans les mêmes conditions que celles décrites précédemment pour le glucose, le fructose et le D-allulose :

- en réalisant une hydrolyse des dimères glucose-glucose de l'échantillon ;
- en déterminant la quantité de glucose total dans le même chromatographe et dans les mêmes conditions, le dit glucose total comprenant le glucose initial dit libre et le glucose issu de l'hydrolyse des dimères glucose-glucose ;
- en soustrayant, à cette quantité de glucose total, la quantité de glucose initial de l'échantillon.

La quantité totale de dimères peut quant à elle être déterminée dans un chromatographe en phase gazeuse dans les mêmes conditions que décrites précédemment, à la différence que la colonne utilisée est une colonne DB1 capillaire de 30 mètres, présentant un diamètre interne de 0,32 mm et une épaisseur de film de 0,25 $\mu$m et la température de la colonne est programmée de manière suivante : de 200°C jusqu'à 280°C à raison de 5°C/min, maintien à 280°C pendant 6 min, puis de 280 °C jusqu'à 320°C à 5°C/min, maintien à 320°C pendant 5 min.

La méthode est décrite plus en détail dans la partie Exemples.

Pour réaliser l'étape de nanofiltration utile à l'invention, la composition à nanofiltrer est passée sur une membrane de nanofiltration. Elle présente généralement une matière sèche allant de 5 à 15%.

La température de cette composition à nanofiltrer peut aller de 10 à 80°C, généralement de 15 à 50°C, souvent aux alentours de 20°C.

L'Homme du métier saura choisir la membrane utile à cette séparation. Cette membrane de nanofiltration a un seuil de coupure inférieur à 300 Da, de préférence allant de 150 à 250 Da. Idéalement, la membrane a un taux de réjection de MgSO4 d'au moins 98%. Il peut notamment s'agir d'une membrane de type Dairy DK ou Duracon NF1 fabriquées par GE®.

La pression appliquée à la membrane peut également varier largement et peut aller de 1 à 50 bars, de préférence de 5 à 40 bars, tout préférentiellement de 15 à 35 bars.

Cette étape de nanofiltration peut être accompagnée d'une phase de diafiltration.

De préférence, le facteur de concentration volumique (FCV) de la nanofiltration va de 2 à 20. Ce facteur de concentration volumique est aisément réglé par l'Homme du métier.

Cette étape de nanofiltration peut être réalisée en continu.

Selon l'invention, l'étape de fourniture d'une composition riche en D-allulose peut comprendre :

- une étape de fourniture d'une composition comprenant du D-fructose (Flux 1 ou 1') ;
- une étape d'épimérisation pour former une composition comprenant du D-fructose et du D-allulose (Flux 2) ;
- une étape de chromatographie pour fournir une composition riche en D-allulose (Flux 5) et un raffinat, qui est une composition riche en D-fructose (Flux 14).

De préférence, l'étape de nanofiltration est réalisée entre l'étape de fourniture de la composition riche en D-allulose (Flux 5) et l'étape de concentration pour fournir la solution mère de D-allulose (Flux 7). L'étape de nanofiltration de ladite composition riche en D-allulose fournit un rétentat (Flux 12) et un perméat (Flux 6).

Ainsi, le procédé selon l'invention comprend:

- une étape de fourniture d'une composition riche en D-allulose ;
- une étape de nanofiltration de ladite composition riche en D-allulose pour fournir un rétentat et un perméat, l'étape de nanofiltration étant réalisée avec une membrane ayant un seuil de coupure inférieur à 300 Da, de préférence allant de 150 à 250 Da ;
- une étape de récupération du perméat de nanofiltration ;
- une étape de concentration de ce perméat pour fournir la solution mère de D-allulose ;

- une étape de cristallisation de la solution mère pour former des cristaux de D-allulose et des eaux mères.

Dans le procédé de l'invention, l'étape de nanofiltration est ainsi avantageusement réalisée sur la composition riche en D-allulose issue de l'étape de chromatographie, juste avant l'étape de concentration qui fournit la solution mère. C'est dans cette configuration que le procédé permet de limiter de manière la plus efficace la quantité de dimères de D-allulose dans la solution mère à cristalliser, et d'augmenter donc de manière la plus importante le rendement global en cristaux de D-allulose.

Par composition riche en D-allulose, on entend généralement une composition présentant en masse sèche une teneur massique de D-allulose supérieure à 80%, avantageusement allant de 80 à 99%, préférentiellement de 82 à 98%.

En ce qui concerne le perméat obtenu, sa matière sèche peut varier, par exemple dans la gamme allant de 3 à 15%. Le perméat peut notamment comprendre, en plus du D-allulose, du D-fructose et du glucose, ainsi que d'autres sucres éventuellement présents. La composition de ce filtrat peut être très différente et dépend de la composition à nanofiltrer. A l'issue de cette étape de nanofiltration, le perméat récupéré peut comprendre, par rapport à sa masse sèche, de 0 à 1,2% de dimères de D-allulose, par exemple de 0,1 à 1,0%, notamment de 0,15 à 0,5%. Le perméat peut être soumis, à une ou plusieurs étapes telles qu'une étape de mélange avec un produit additionnel, une étape de séparation, une étape de purification, une étape d'épimérisation ou une étape de concentration.

En ce qui concerne le rétentat obtenu, sa matière sèche peut également varier largement, par exemple dans la gamme allant de 15 à 40%. Il peut comprendre principalement du D-fructose, du D-allulose, du glucose et des dimères de D-allulose. Selon une variante, le rétentat est récupéré, éventuellement mélangé avec une composition additionnelle de D-allulose, pour fournir, après une étape de concentration éventuelle, un sirop de D-allulose.

Dans la variante préférée où la composition riche en D-allulose qui comprend des dimères de D-allulose est soumise à une étape de nanofiltration, le perméat obtenu (Flux 6), dit « perméat préféré », comprend de préférence en masse sèche :

- de 80 à 99% de D-allulose ;
- de 0 à 20% de D-fructose ;
- de 0 à 10% de glucose ;
- de 0 à 1,2% de dimères de D-allulose.

Comme indiqué ci-dessus, ce perméat préféré peut être directement soumis à une étape de concentration pour obtenir la solution mère à cristalliser (Flux 7).

Il est précisé que dans la présente Demande, hormis les cristaux de D-allulose, l'ensemble des compositions sont généralement des compositions aqueuses. En d'autres termes, le solvant des constituants secs comprend de l'eau. Le solvant des compositions est généralement constitué d'eau ou d'un mélange d'eau et d'alcool tel que, par exemple, l'éthanol. Préférentiellement, le solvant des compositions est de l'eau.

Ainsi, la solution mère de D-allulose utile à l'invention consiste généralement en une solution aqueuse de D-allulose. La solution mère présente généralement une matière sèche d'au moins 75%, par exemple de 80 à 95%, préférentiellement de 81 à 92%, tout préférentiellement de 83 à 89%.

Pour atteindre cette matière sèche, il est nécessaire de réaliser une étape de concentration. Cette étape peut être réalisée sur une composition riche en D-allulose, la seule exigence est que cette composition riche en D-allulose ait été obtenue par un procédé comprenant, dans une étape antérieure, l'étape de nanofiltration utile à l'invention. Cette composition riche en D-allulose soumise à l'étape de concentration peut ainsi être le perméat préféré décrit précédemment, mais également une composition riche en D-allulose obtenue par chromatographie, ou encore un mélange d'un perméat avec une composition riche en D-allulose additionnelle. Préférentiellement, la composition riche en D-allulose soumise à l'étape de concentration est le perméat préféré.

La formation de dimères de D-allulose se produisant également lors de l'étape de concentration, il est préférable de sélectionner des conditions permettant de limiter les quantités formées en ces dimères. L'étape de concentration est ainsi généralement réalisée sous vide, par exemple à une pression de 5 à 100 mbars, de préférence allant de 20 à 70mbars. Ce vide permet de diminuer la température nécessaire à l'évaporation et de réduire la durée de cette étape de concentration. Elle peut être réalisée à une température allant de 30 à 80°C, avantageusement de 34 à 70°C, préférentiellement de 37 à 50°C. Cette étape de concentration peut être réalisée dans un évaporateur simple étage, un évaporateur multiples étages, par exemple un évaporateur double étages. A l'issue de l'étape de concentration, la solution mère de D-allulose utile à l'invention est obtenue.

Cette étape de concentration peut être réalisée en continu.

La solution mère obtenue peut comprendre en masse sèche :

- de 80 à 99% de D-allulose, de préférence de 85 à 98% ;
- de 0 à 20% de D-fructose, de préférence de 0,5 à 15% ;

- de 0 à 10% de glucose, de préférence de 0 à 5% ;
- de 0 à 1,5% de dimères de D-allulose, par exemple de 0,1 à 1,2%, préférentiellement de 0,4 à 1,1%.

Le procédé selon l'invention comprend en outre une étape de cristallisation de ladite solution mère de D-allulose pour former une suspension de cristaux de D-allulose. Cette suspension comprend des cristaux et eaux mères de cristallisation qui sont également formées lors de cette étape.

Cette étape de cristallisation peut être de tout type. Il peut notamment s'agir d'une étape de cristallisation par refroidissement ou une étape de cristallisation par évapocristallisation. L'Homme du métier saura trouver les conditions de mise en œuvre qui sont notamment décrites dans le document WO 2011/119004. Toutefois, il faut noter que les étapes de cristallisation décrites dans ces documents sont rendues plus faciles à réaliser du fait de la préparation particulière de la solution mère utile à l'invention.

A l'issue de l'étape de cristallisation, à partir de la suspension de cristaux (Flux 9) les cristaux (Flux 10) sont séparés des eaux mères (Flux 13), notamment par une étape de filtration et/ou de centrifugation. Cette étape de séparation se fait plus préférentiellement en batch.

Les eaux mères (Flux 13) présentent généralement une matière sèche allant de 70 à 80%. Elles peuvent comprendre en masse sèche :

- de 80 à 99% de D-allulose, de préférence de 82 à 95% ;
- de 0 à 20% de D-fructose, de préférence de 0,5 à 15% ;
- de 0 à 10% de glucose, de préférence de 0 à 5% ;
- de 0 à 3% de dimères de D-allulose, par exemple de 0,1 à 2,9%, notamment de 1 à 2,5%.

Les cristaux obtenus peuvent être soumis à une étape de clairçage à l'eau froide et/ou à l'alcool, notamment l'éthanol. Ces cristaux peuvent être ensuite séchés (Flux 11) par une étape de séchage qui peut se faire dans tout type de séchoir adapté. Les cristaux de D-allulose présentent une teneur en eau inférieure à 5%, de préférence inférieure à 1%.

Cette étape de cristallisation peut être réalisée en continu, notamment à l'aide d'un cristallisoir vertical, dont un exemple est représenté à la Figure 5.

Selon un mode tout préféré, l'étape de cristallisation comprend un stade d'évapo-refroidissement adiabatique, réalisé dans un cristallisoir-évaporateur adiabatique sous vide pour former une masse-cuite (Flux 8), suivi d'un stade de cristallisation par refroidissement de ladite masse-cuite pour fournir une suspension de cristaux (Flux 9). Un évapo-refroidissement adiabatique provoque un refroidissement imméidat de la solution mère à cristalliser. De préférence, le cristallisoir-évaporateur est équipé d'un condenseur et l'eau condensée lors de ce stade est réinjectée en continu le long des parois en haut du cristallisoir pour maintenir stable la matière sèche. Cette étape de cristallisation préférée comprenant deux stades distincts, combinée à l'étape de nanofiltration dans une configuration particulière du procédé de l'invention, a permis d'obtenir de manière continue les cristaux de l'invention qui sont décrits plus bas dans la description. Ceci est lié au fait que cette étape de cristallisation préférée, notamment le stade d'évapo-refroidissement, permet également de limiter de manière très importante la formation in-situ de dimères de D-allulose. Sans être liée à une quelconque théorie, la Demanderesse l'explique par le fait que la solution mère peut être refroidie de manière quasiment instantanée lors de son introduction dans le cristallisoir-évaporateur, à la différence des procédés de cristallisation déjà connus du D-allulose qui décrivent le refroidissement naturel de la solution mère, voire par une rampe de refroidissement à l'aide d'un échangeur de chaleur comme décrit dans la demande WO2016/064087. Les cristaux selon l'invention présentent une pureté et des propriétés améliorées, bien que le premier stade de l'étape de cristallisation soit un stade d'évapo-refroidissement instantané, ce qui raccourcit la durée de l'étape de cristallisation. Or, ceci est contraire à ce qu'aurait envisagé l'Homme du métier, pour qui l'obtention de cristaux améliorés demande une durée de cristallisation augmentée.

Lors du stade d'évapo-refroidissement, la température peut aller de 30 à 40°C, de préférence allant de 33 à 37°C, par exemple d'environ 35°C. Cette température est aisément atteinte par l'Homme du métier en déterminant la dépression adéquate à appliquer. Ainsi, la pression dans le cristallisoir-évaporateur peut aller de 30 à 50 mbar. Un cristallisoir adiabatique peut notamment être un cristallisoir à tube de tirage de type DT (pour *Draft Tube)* ou de type DTB (pour *Draft Tube Baffle),* à circulation forcée ou à circulation forcée indirecte (IFC®). De préférence, ce stade d'évapo-refroidissement est continu. Lors de ce stade, les amorces sont générées en continu par phénomène de nucléation spontanée dans l'évapo-cristalllisoir par la sursaturation créée par le refroidissement rapide ; ainsi, il n'y a pas à proprement parler d'introduction d'amorces dans ce cas. A titre non limitatif, une possibilité de réaliser ce stade d'évapo-refroidissement en continu est décrite dans la partie Exemples et à la Figure 4, où une fraction des cristaux formés lors du stade d'évapo-refroidissement (Flux 7d) est mélangée dans le Flux 7b d'alimentation du cristallisoir-évaporateur, ce qui permet d'obtenir un Flux 7c qui comprend, au moment de l'introduction dans le cristallisoir-évaporateur, des cristaux qui vont pouvoir encore grossir lors de ce nouveau passage dans le cristallisoir-évaporateur. Selon cet exemple, le Flux 7b peut être obtenu à partir d'un mélange du Flux 7 et du Flux 7a, qui consiste en un sirop sursaturé de D-allulose récupéré dans le cristallisoir-évaporateur, qui comprend des « fines », c'est-à-dire les cristaux les plus fins de D-allulose du cristallisoir. Selon cette variante, le Flux 7 peut avantageusement, avant mélange, passer dans un échangeur de chaleur, ce passage permettant de réchauffer de manière quasiment immédiate le Flux 7 avant de le mélanger immédiatement avec le Flux 7a. Ceci permet alors de refondre les fines du Flux 7a et refroidir le Flux 7 pour obtenir un Flux 7b exempt de ces fines.

A l'issue de ce stade, on récupère une masse-cuite de cristaux de D-allulose (Flux 8), c'est-à-dire une suspension de cristaux, présentant généralement une petite taille. Le temps de séjour moyen de la masse-cuite lors de ce stade d'évaporefroidissement peut être compris entre 5 et 15 heures. La taille moyenne en volume D4,3 des cristaux en suspension dans la masse-cuite va généralement de 50 à 200 $\mu$m.

Le stade de cristallisation par refroidissement se fait de manière classique en refroidissant la masse-cuite obtenue lors du stade d'évapo-refroidissement (Flux 8). La durée de ce stade de cristallisation peut aller de 25 à 50 heures. La température de début de cristallisation dépend généralement de la température de la masse-cuite introduite et peut notamment aller de 30 à 40°C, de préférence allant de 33 à 37°C, par exemple d'environ 35°C. Ce stade se fait généralement sous agitation mécanique. De préférence, lors de la cristallisation par refroidissement, la température est diminuée à une vitesse allant de 0,3 à 0,5°C par heure. La Figure 5 représente un exemple de cristallisoir vertical avec, sur les côtés, différents échangeurs thermiques permettant de régler la température dans le cristallisoir. Lors de cette opération, on préfère que la différence de température entre la masse-cuite et l'eau de l'échangeur n'excède pas 5°C. Le stade de cristallisation par refroidissement peut être de préférence réalisé en continu, notamment dans un cristallisoir vertical.

Ainsi, une variante préférée du procédé de l'invention comprend :

- une étape de fourniture d'une composition riche en D-allulose (Flux 5) ;
- une étape de nanofiltration de ladite composition riche en D-allulose pour fournir un rétentat (Flux 12) et un perméat (Flux 6) ;
- une étape de récupération du perméat de nanofiltration ;
- une étape de concentration de ce perméat pour fournir la solution mère de D-allulose (Flux 7) ;
- une étape de cristallisation comprenant :

    i. un stade d'évapo-refroidissement adiabatique, réalisé dans un cristallisoir-évaporateur adiabatique sous vide pour former une masse-cuite (Flux 8),
    ii. suivi d'un stade de cristallisation par refroidissement de ladite masse-cuite pour former une suspension de cristaux (Flux 9).

Hormis les avantages liés aux cristaux eux-mêmes, un avantage de cette variante préférée du procédé de l'invention est que les cristaux obtenus peuvent être encore plus aisément séparés des eaux mères de cristallisation et plus facilement séchés. Ceci est principalement lié à la forme des cristaux obtenue.

Pour démarrer une étape de cristallisation, des amorces de D-allulose sont généralement introduites dans le cristallisoir sélectionné. Ces amorces de D-allulose consistent en des cristaux de D-allulose de petite taille, présentant par exemple une taille allant de 10 à 100 $\mu$m. La quantité massique d'amorce peut varier largement selon le type de cristallisoir utilisé. Elle peut aller de 0,001 à 1%, souvent de 0,01 à 0,7%, généralement de 0,05 à 0,5% par rapport à la masse de D-allulose dans la solution mère. Ces quantités sont particulièrement adaptées lorsque l'on réalise une étape de cristallisation par refroidissement à partir d'une solution mère de D-allulose. Comme évoqué ci-dessus, il est également possible de créer des amorces in situ lorsque l'on utilise une étape d'évapo-refroidissement.

De préférence, l'étape de cristallisation est réalisée moins d'une heure après l'étape de concentration, de préférence moins de 30 minutes après. Tout préférentiellement, l'étape de cristallisation est réalisée immédiatement après l'étape de concentration. Ceci permet de limiter encore, au moment de l'étape de cristallisation, la quantité de dimères dans la solution mère à cristalliser.

Une fois récupérés après séchage, les cristaux peuvent également être soumis à une étape supplémentaire de tamisage, qui permet de cribler ces cristaux et, selon la fraction récupérée après tamisage, d'augmenter ou diminuer la taille des cristaux. Par exemple, cette étape supplémentaire permet, par rapport à la taille des cristaux soumis à l'étape de tamisage, de récupérer une fraction de plus petite taille moyenne en volume D4,3 de cristaux de D-allulose passée à travers le tamis ainsi qu'une fraction de plus grande taille moyenne en volume D4,3 de cristaux de D-allulose restant dans le tamis. Pour modifier la population de cristaux et obtenir la fraction D 4,3 désirée, il suffit à l'Homme du métier de sélectionner la taille des mailles du tamis.

Il va sans dire que le procédé selon l'invention peut comprendre d'autres étapes, telles que les autres étapes figurant dans le procédé classique décrit précédemment et qui vont être décrites en détail par la suite. Le procédé selon l'invention peut également comprendre des étapes additionnelles de purification et également des étapes de dilution ou de concentration intermédiaires en vue de régler matière sèche et ainsi réaliser dans les meilleures conditions les différentes étapes du procédé de l'invention. L'ensemble de ces étapes peuvent être réalisées en continu.

La composition de D-fructose fournie (Flux 1) pour réaliser l'étape d'épimérisation peut être un sirop de D-fructose, qui peut être obtenu par dissolution de cristaux de D-fructose dans de l'eau ou un sirop de glucose/D-fructose. Préférentiellement, cette composition comprend un sirop de glucose/D-fructose qui comprend au moins 90% en poids sec de D-fructose, préférentiellement au moins 94% de fructose. Dans un mode préféré qui sera explicité plus loin dans la description, la composition de D-fructose fournie pour réaliser l'étape ultérieure d'épimérisation est un mélange (Flux 1') de ce sirop de D-fructose avec au moins une fraction recyclée qui peut être le raffinat en tout ou partie (Flux 14 ou 16), cette fraction recyclée pouvant comprendre une quantité de D-allulose supérieure.

La composition de D-fructose soumise à l'étape d'épimérisation peut comprendre :

- de 0 à 10% de D-allulose ;
- de 70 à 100% de D-fructose ;
- de 0 à 10% de glucose ;
- de 0 à 15% de dimère de D-allulose.

L'étape d'épimérisation est réalisée à partir de la composition de D-fructose fournie précédemment, éventuellement après réglage de la matière sèche. Cette étape est généralement réalisée à une matière sèche allant de 30 à 60%, souvent de 45 à 55%. On introduit dans cette composition une enzyme de type D-psicose epimerase ou une composition comprenant cette enzyme. La composition comprenant cette enzyme peut être un lyophilisat d'un microorganisme hôte synthétisant la D-psicose epimerase, celui-ci pouvant être le *Bacillus subtilis,* notamment celui décrit dans la demande WO2015/032761 A1. Le pH est réglé selon l'enzyme utilisée, par exemple à un pH allant de 5,5 à 8,5. La réaction peut être réalisée en chauffant à une température allant de 40 à 70°C, souvent de 45 à 60°C. La réaction peut durer de 0,1 à 100 heures, par exemple de 0,2 à 60 heures. Cette réaction peut par exemple se faire sur une colonne enzymatique, ce qui présente l'avantage de travailler également en continu sur cette étape. Il est également possible pour fonctionner en continu de travailler séquentiellement avec plusieurs réacteurs. Pour réaliser cette étape d'épimérisation, on peut notamment utiliser l'enseignement du document WO 2015/032761 A1.

A l'issue de la réaction est formée une composition comprenant du D-fructose et du D-allulose, généralement selon un ratio massique D-fructose / D-allulose allant de 85/15 à 55/45, souvent selon un ratio massique D-fructose / D-allulose allant de 80/20 à 60/40. Ce ratio dépend des paramètres d'épimérisation utilisés et, bien évidemment, de la quantité de D-allulose et de D-fructose dans la composition de D-fructose fournie dans l'étape d'épimérisation ; la quantité de D-allulose dans cette composition peut être notamment plus importante en cas de recyclage.

A l'issue de cette étape d'épimérisation, si nécessaire, une étape de filtration peut être réalisée pour récupérer les débris cellulaires éventuellement présents, notamment lorsqu'un lyophilisat d'un microorganisme hôte est utilisé. Cette étape peut consister en une étape de microfiltration.

Dans la Figure 3, la composition microfiltrée correspond au Flux 3 et les débris cellulaires sont récupérés dans le Flux 17.

Dans le procédé de l'invention, des étapes de purifications additionnelles peuvent également être réalisées. On réalise généralement, avant l'étape de chromatographie, une étape de déminéralisation de la composition comprenant du D-fructose et du D-allulose (Flux 3) qui peut être réalisée par passage sur une ou plusieurs résines échangeuse d'ions cationiques (par exemple une résine cationique de type Dowex 88), anioniques (par exemple une résine anionique de type Dowex 66) et un mélange cationique-anionique. Dans la Figure 3, cette composition correspond au Flux 4. La composition comprenant du D-fructose et du D-allulose obtenue est alors déminéralisée et présente généralement une résistivité supérieure à 100 kΩ.cm$^{-1}$. On peut également réaliser, avant cette étape de déminéralisation, une étape de décoloration de la composition comprenant du D-fructose et du D-allulose, par exemple en passant sur une colonne comprenant du charbon actif.

La composition comprenant du D-fructose et du D-allulose (Flux 4) peut ensuite être soumise à une étape de chromatographie pour fournir au moins une composition riche en D-allulose et une composition riche en D-fructose. Dans un mode préféré qui sera explicité en détail plus loin dans la description, la composition comprenant du D-fructose et du D-allulose soumise à l'étape de chromatographie est un mélange (Flux 4') de la composition issue de l'étape d'épimérisation (Flux 4) et d'au moins une fraction recyclée, cette fraction recyclée pouvant comprendre une quantité de D-allulose supérieure.

La composition soumise à l'étape de chromatographie peut comprendre, par rapport à sa masse sèche :

- de 22 à 45% de D-allulose, généralement de 25 à 37% ;
- de 45 à 75% de D-fructose, généralement de 46 à 70% ;
- de 0 à 10% de glucose ;
- de 2 à 10% de dimère de D-allulose.

Pour réaliser cette étape de chromatographie, on peut utiliser tout type de chromatographie continue, notamment de type chromatographie à lit mobile simulé *(Simulated Moving Bed SMB)*, de type *Improved Simulated Moving Bed (ISMB)*, de type *Divide Improved Simulated Moving Bed (DISMB), de type Sequential Simulated Moving Bed (SSMB)* ou de type *Nippon Mitsubishi Chromatography Improved (NMCI)*. On utilise généralement de l'eau comme éluant. La chromatographie peut être équipée de plusieurs colonnes en série par exemple de 4 à 8 colonnes. Les colonnes comprennent de la résine échangeuse d'ions, par exemple une résine cationique échangeuse d'ions calcium. La matière sèche de la composition comprenant du D-fructose et du D-allulose peut aller de 40 à 70%, généralement est d'environ 50%. La température de la composition lors de la chromatographie va généralement de 40 à 80°C, de préférence de 55 à 65°C. Cette chromatographie dure le temps d'obtenir une séparation satisfaisante et peut durer plusieurs heures.

On obtient à l'issue de cette étape une composition riche en D-allulose (Flux 5) qui peut comprendre, par rapport à sa matière sèche, au moins 80% de D-allulose, avantageusement au moins 90% de D-allulose. Cette composition riche en D-allulose peut présenter une matière sèche allant de 5 à 15%. On obtient également à l'issue de cette étape un raffinat (Flux 14), qui comprend généralement par rapport à sa matière sèche au moins 75% de D-fructose, souvent au moins 80% de D-fructose. Ce raffinat présente généralement une matière sèche allant de 15 à 30% environ.

La composition riche en D-allulose obtenue à l'issue de la chromatographie (Flux 5) peut ainsi comprendre, par rapport à sa masse sèche :

- de 80 à 98% de D-allulose ;
- de 0 à 20% de D-fructose ;
- de 0 à 10% de glucose ;
- de 1,5 à 5% de dimère de D-allulose.

Le raffinat peut comprendre quant à lui, par rapport à sa masse sèche :

- de 1 à 10% de D-allulose ;
- de 70 à 99% de D-fructose ;
- de 0 à 10% de glucose ;
- de 5 à 20% de dimère de D-allulose.

Contrairement à un procédé classique où, par rapport au D-fructose introduit, le rendement global en cristaux de D-allulose est inférieur à 15%, le rendement du procédé de l'invention peut être supérieur ou égal à 25%. Avantageusement, le rendement global en cristaux de D-allulose est supérieur ou égal à 50%, par exemple supérieur ou égal à 60%, voire supérieur ou égal à 65%. Ce rendement particulièrement amélioré est rendu possible par le fait qu'il est possible d'effectuer des étapes de recyclage sans perturber l'étape de cristallisation.

Ainsi le procédé de l'invention peut comprendre au moins une étape de recyclage.

Selon un mode préféré, cette étape de recyclage peut consister en une étape de recyclage d'au moins une partie du raffinat issu de l'étape de chromatographie (Flux 14). Ce raffinat peut éventuellement avoir été concentré avant d'être mélangé. Il peut avantageusement être recyclé, en tout ou partie, pour être mélangé à du D-fructose (Flux 1), par exemple sous la forme du sirop de D-fructose glucose décrit précédemment, pour fournir la composition de D-fructose (Flux 1'). C'est alors cette composition de D-fructose, qui est généralement plus riche en D-allulose que le sirop de D-fructose, qui est soumise à l'étape d'épimérisation.

Cette étape de recyclage peut consister en une étape de recyclage d'au moins une partie des eaux mères (Flux 13). Ces eaux mères peuvent avantageusement être recyclées, en tout ou partie, pour être mélangées à la composition comprenant du D-fructose et du D-allulose (Flux 4). C'est alors ce mélange (Flux 4') qui est soumis à l'étape de chromatographie. Ces eaux mères peuvent éventuellement avoir été diluées avant d'être mélangées.

Cette étape de recyclage peut consister en une étape de recyclage d'au moins une partie du rétentat (Flux 12). Ce rétentat peut avantageusement être recyclé, en tout ou partie, pour être mélangé à la composition comprenant du D-fructose et du D-allulose (Flux 4) et éventuellement les eaux mères recyclées. C'est alors ce mélange (Flux 4') qui est soumis à l'étape de chromatographie. Ce rétentat peut éventuellement avoir été concentré avant d'être mélangé. Dans le cas où le mélange se fait avec les fractions rétentat et eaux-mères, il peut ne pas être nécessaire de concentrer ou diluer ces fractions. Il faut noter que les recyclages des fractions rétentat et eaux-mères, qui sont deux fractions présentant des quantités relativement importantes de D-allulose, permettent d'augmenter la richesse en D-allulose (et par conséquent diminuer la quantité de D-fructose) de la composition soumise à l'étape de chromatographie. Pour réaliser les étapes éventuelles de concentration des fractions recyclées, il est possible d'utiliser les mêmes équipements et conditions décrits pour l'étape de concentration permettant la fabrication de la solution mère.

Selon un mode tout préféré de l'invention (la Figure 3 représente un circuit de production à ce procédé préféré de l'invention), le procédé comprend :

a) une étape de fourniture d'une composition comprenant du D-fructose (Flux 1') ;

b) une étape d'épimérisation pour former une composition comprenant du D-fructose et du D-allulose (Flux 2) ;

c) une étape de chromatographie pour fournir une composition riche en D-allulose (Flux 5) et un raffinat consistant en une composition riche en D-fructose (Flux 14) ;

d) une étape de nanofiltration de la composition riche en D-allulose pour former un perméat (Flux 6) et un rétentat (Flux 12) ;

e) une étape de concentration du perméat pour former la solution mère à cristalliser (Flux 7) ;

f) une étape de cristallisation de la solution mère (Flux 9) pour former des cristaux (Flux 10) et des eaux mères (Flux 13) ;

et dans lequel est réalisée :

- une étape de recyclage d'au moins une partie du raffinat (Flux 14 ou 16) pour être mélangé avec du D-fructose (Flux 1) et fournir la composition (Flux 1') de l'étape a) ;
- et/ou une étape de recyclage d'au moins une partie du rétentat (Flux 12) issu de l'étape de nanofiltration pour être mélangé avec la composition comprenant du D-fructose et du D-allulose (Flux 4) issue de l'étape b) pour fournir la composition (Flux 4') soumise à l'étape de chromatographie c);
- et/ou une étape de recyclage d'au moins une partie des eaux mères (Flux 13) issues de l'étape de cristallisation pour être mélangées avec la composition comprenant du D-fructose et du D-allulose issue de l'étape b) (Flux 4) pour fournir la composition (Flux 4') soumise à l'étape de chromatographie c).

Le procédé comprend une étape de purge, cette étape de purge pouvant être une étape de purge d'au moins une partie d'au moins une des fractions recyclées choisies parmi le raffinat, le rétentat et les eaux mères de cristallisation. En effet, pour que le système reste stable, il est absolument nécessaire de retirer du circuit de production une partie des dimères de D-allulose formés lors du procédé. De manière générale, plus les fractions sont recyclées, plus la quantité de dimères de D-allulose augmente dans le circuit. Ainsi, une possibilité de diminuer la quantité de dimères de D-allulose est d'augmenter les quantités purgées. Toutefois, ceci se fait alors au détriment du rendement global en cristaux de D-allulose. Le procédé de l'invention permet d'augmenter de manière drastique le recyclage des différentes fractions obtenues dans le procédé, tout en maintenant possible la cristallisation.

A titre d'exemple, dans la variante du procédé tout préféré décrit ci-dessus où l'ensemble des fractions raffinat, rétentat et eaux mères de cristallisation sont recyclées, l'étape de recyclage du raffinat est avantageusement un recyclage partiel, par exemple de 50 à 95% de ce raffinat est recyclé (Flux 16) et de 5 à 50% est purgé (Flux 15) pour fournir une composition de D-fructose qui comprend des dimères de D-allulose : en d'autres termes, le taux de recyclage de la composition riche en D-fructose va de 50 à 95%. Préférentiellement, le taux de recyclage va de 70 à 92 %. Dans ce cas, les deux autres recyclages sont avantageusement des recyclages totaux.

Les fractions purgées peuvent ensuite être utilisées pour fabriquer des sirops, éventuellement après une étape de concentration et/ou de mélange avec d'autres compositions et/ou d'additifs. Du fait que l'étape de cristallisation reste stable au cours du temps, le procédé selon l'invention est particulièrement avantageux car il peut être continu.

Comme expliqué précédemment, selon la variante préférée de l'invention combinant l'étape de nanofiltration et de cristallisation dans un cristallisoir-évaporateur adiabatique sous vide, la Demanderesse est également parvenue à obtenir des cristaux présentant une qualité améliorée. Les cristaux selon l'invention présentent une teneur massique en dimère de D-allulose inférieure à 0,50%, de préférence inférieure à 0,30%, voire inférieure à 0,20%. Ces cristaux peuvent avantageusement comprendre une teneur massique en dimère de D-allulose allant de 0,01 à 0,48%, préférentiellement allant de 0,02 à 0,45%, par exemple allant de 0,03 à 0,40%, notamment de 0,04 à 0,30%, particulièrement de 0,05 à 0,20%.

Les cristaux selon la présente divulgation présentent avantageusement une teneur massique en D-allulose supérieure ou égale à 99,00%, de préférence supérieure ou égale à 99,50%, voire supérieure ou égale à 99,70%.

Un avantage des cristaux de l'invention est que ces cristaux présentent de faibles quantités de dimère de D-allulose. Sans être liée par une quelconque théorie, la Demanderesse l'explique par le fait que la solution mère utile à la fabrication de ces cristaux comprend une teneur en dimères de D-allulose très faible et par le fait que l'étape de cristallisation mise en œuvre permet de limiter la formation de ces dimères in-situ. Ces cristaux ont ainsi pu être obtenus par la Demanderesse par ce procédé, en combinant l'étape de nanofiltration avec l'étape de cristallisation comprenant un stade d'évapo-refroidissement adiabatique et un stade de cristallisation par refroidissement.

Un désavantage associé aux cristaux de D-allulose de l'art antérieur, qui comprennent une teneur en dimère plus élevée que ceux de l'invention, est que l'on observe un allongement de ces cristaux pour présenter une forme proche d'aiguilles, notamment lorsqu'ils sont de grande taille.

Les cristaux de D-allulose de l'invention présentent une taille moyenne en volume D4,3 supérieure à 200 $\mu$m, avantageusement allant de 210 à 800 $\mu$m, préférentiellement de 220 à 350 $\mu$m.

Les cristaux de l'invention, qui présentent une teneur en dimère de D-allulose plus faible, présentent l'avantage de présenter une forme plus « trapue », comme démontré dans les Figures 11, 12 et 13.

Cette forme plus trapue peut se traduire par le fait que les cristaux de D-allulose de l'invention peuvent présenter, pour une taille de particules en volume D 4,3 donnée et choisie dans la gamme allant de 200 à 400$\mu$m, un rapport de diamètres Feret min/Feret max supérieur à 0,60, avantageusement allant de 0,62 à 0,90, par exemple de 0,63 à 0,80. Le diamètre Feret est une grandeur bien connue de l'Homme du métier. Il est déduit de l'aire projetée d'une particule, en utilisant le principe du pied à coulisse. Le diamètre Feret min consiste en la plus petite des dimensions alors que le diamètre Feret max consiste en la plus grande des dimensions. La Figure 15 représente le principe des diamètres Feret min et max sur une particule donnée.

De préférence, les cristaux de l'invention présentent ce rapport Feret min/Feret max sur l'ensemble des tailles de particules dans la gamme allant de 200 à 400$\mu$m.

De manière alternative et indépendamment de la teneur en dimères de D-allulose, un objet de l'invention porte sur des cristaux de D-allulose qui présentent une taille moyenne en volume D4,3 supérieure à 200 $\mu$m, avantageusement allant de 210 à 800$\mu$m, préférentiellement de 220 à 350 $\mu$m et présentant, pour une taille de particules en volume D 4,3 donnée et choisie dans la gamme allant de 200 à 400$\mu$m, un rapport de diamètres Feret min/Feret max supérieur à 0,60, avantageusement allant de 0,62 à 0,90, par exemple de 0,63 à 0,80. De préférence, les cristaux de l'invention présentent ce rapport Feret min/Feret max sur l'ensemble des tailles de particules dans la gamme allant de 200 à 400$\mu$m. Ces cristaux de l'invention comprennent une teneur massique en dimère de D-allulose inférieure à 0,50%, de préférence inférieure à 0,30%, voire inférieure à 0,20%. Ces cristaux peuvent avantageusement comprendre une teneur massique en dimère de D-allulose allant de 0,01 à 0,48%, préférentiellement allant de 0,02 à 0,45%, par exemple allant de 0,03 à 0,40%, notamment de 0,04 à 0,30%, particulièrement de 0,05 à 0,20%. Ces cristaux présentent avantageusement une teneur massique en D-allulose supérieure ou égale à 99,00%, de préférence supérieure ou égale à 99,50%, voire supérieure ou égale à 99,70%.

La taille moyenne en volume D4,3 ainsi que le rapport de diamètres Feret min/Feret max des cristaux sont déterminés par un granulomètre, notamment le granulomètre de type QICPIC RODOS de la marque SympaTEC tel que celui utilisé dans la partie Exemples. Etant donné que les cristaux sont observés de manière statistique dans toutes les directions dans un granulomètre, les valeurs obtenues par un granulomètre pour une population de cristaux sont différentes des valeurs obtenues par calcul sur un simple cliché microscopique de cette population de cristaux, les valeurs obtenues par granulométrie étant généralement supérieures.

De préférence, les cristaux sont non agglomérés (ou individualisés). Le fait que les cristaux sont non agglomérés peut se vérifier par simple observation par microscopie optique. A titre d'exemple, les cristaux de la Figure 13 sont agglomérés, au contraire de ceux des Figures 11 et 12.

Cette forme différente se traduit à l'échelle macroscopique par un écoulement amélioré des cristaux de l'invention en comparaison avec des cristaux de même taille moyenne. Egalement, ces cristaux peuvent présenter un meilleur comportement au mottage au cours du temps.

Ainsi, du fait de leurs propriétés, les cristaux obtenus par le procédé préféré de l'invention peuvent s'écouler facilement. C'est ainsi qu'ils ont permis l'obtention de cristaux présentant un écoulement jamais atteint à ce jour. Ils peuvent ainsi être par exemple utilisés avantageusement comme sucre de table.

Les cristaux de D-allulose de l'invention peuvent être utilisés dans les applications connues de l'allulose et, de manière générale, des édulcorants. Parmi les applications pouvant utiliser les cristaux de D-allulose de l'invention peuvent être citées les chewings-gums sous forme de tablettes ou dragées, les bonbons et les comprimés à sucer, les biscuits, les cookies, les muffins, les gâteaux, les gâteaux à base de gélatine, les pâtes à mâcher notamment les pâtes à mâcher à texture courte, les glaçages et les boissons en poudre.

Un autre objet de l'invention porte également sur l'utilisation d'une unité de nanofiltration dans un circuit de production de cristaux de D-allulose pour améliorer le rendement global en cristaux de D-allulose. Cette utilisation est particulièrement avantageuse lorsque la matière première introduite dans le circuit comprend du D-fructose.

Un autre objet de la présente divulgation porte également sur l'utilisation d'une unité de nanofiltration dans un circuit de production de cristaux de D-allulose pour améliorer la qualité des cristaux obtenus. Par améliorer la qualité des cristaux obtenus, on entend notamment diminuer la teneur en dimère de D-allulose et/ou augmenter le rapport de diamètres Feret min/Feret max des cristaux de D-allulose.

A titre illustratif, d'autres modes de réalisation du procédé selon l'invention, comprenant une étape de recyclage des eaux mères et/ou une étape de recyclage du raffinat et/ou une étape de recyclage du rétentat sont présentés ci-après.

Selon un premier mode de réalisation ne faisant pas partie de la présente invention, le procédé comprend :

a) une étape de fourniture d'une composition riche en D-allulose ;

b) une étape de concentration pour former la solution mère à cristalliser ;

caractérisé en ce que :

- au moins une étape de recyclage consiste en une étape de recyclage d'au moins une partie des eaux mères ;
- l'étape de nanofiltration est réalisée sur ces eaux mères recyclées pour former un perméat un rétentat ;
- le perméat est mélangé avec la composition riche en D-allulose fournie à l'étape a) ; et
- ce mélange est soumis à l'étape de concentration b) pour fournir la solution mère à cristalliser ;
- au moins une partie des eaux mères et/ou du rétentat est purgée.

Selon un deuxième mode de réalisation, le procédé comprend :

a) une étape de fourniture d'une composition riche en D-allulose ;
b) une étape de concentration pour former la solution mère à cristalliser ;

caractérisé en ce que :

- au moins une étape de recyclage consiste en une étape de recyclage d'au moins une partie des eaux mères ;
- ces eaux mères recyclées sont mélangées avec la composition riche en D-allulose fournie à l'étape a) ;
- l'étape de nanofiltration est réalisée sur ce mélange pour fournir un perméat et un rétentat ;
- ce perméat est soumis à l'étape de concentration b) pour fournir la solution mère à cristalliser ; et
- au moins une partie des eaux mères est purgée.

Selon un troisième mode de réalisation ne faisant pas partie de la présente invention, le procédé comprend :

a) une étape de fourniture d'une composition comprenant du D-allulose et du D-fructose ;
b) une étape de chromatographie pour fournir une composition riche en D-allulose et un raffinat consistant en une composition riche en D-fructose ;
c) une étape de concentration de la composition riche en D-allulose pour former la solution mère à cristalliser ;

caractérisé en ce que :

- au moins une étape de recyclage consiste en une étape de recyclage d'au moins une partie des eaux mères ;
- l'étape de nanofiltration est réalisée sur ces eaux mères recyclées pour former un perméat et un rétentat ;
- le perméat est mélangé avec la composition fournie à l'étape a) ;
- ce mélange est soumis à l'étape de chromatographie b) ; et
- au moins une partie des eaux mères est purgée.

Selon un quatrième mode de réalisation ne faisant par partie de la présente invention, le procédé comprend :

a) une étape de fourniture d'une composition comprenant du D-allulose et du D-fructose ;
b) une étape de chromatographie pour fournir une composition riche en D-allulose et une composition riche en D-fructose ;
c) une étape de concentration pour former la solution mère à cristalliser ;

caractérisé en ce que :

- au moins une étape de recyclage consiste en une étape de recyclage d'au moins une partie des eaux mères ;
- ces eaux mères recyclées sont mélangées avec la composition fournie à l'étape a) ;
- l'étape de nanofiltration est réalisée sur ce mélange pour fournir un perméat et un rétentat ;
- ce perméat est soumis à l'étape de chromatographie b) ; et
- au moins une partie des eaux mères est purgée.

Selon un cinquième mode de réalisation, le procédé comprend :

a) une étape de fourniture d'une composition comprenant du D-allulose et du D-fructose ;
b) une étape de chromatographie pour fournir une composition riche en D-allulose et une composition riche en D-fructose ;
c) une étape de concentration pour former la solution mère à cristalliser ;

caractérisé en ce que :

- au moins une étape de recyclage consiste en une étape de recyclage d'au moins une partie des eaux mères ;
- ces eaux mères recyclées sont mélangées avec la composition fournie à l'étape a) ;
- l'étape de chromatographie b) est réalisée sur ce mélange ;
- l'étape de nanofiltration est réalisée sur la composition riche en D-allulose résultant de cette étape de chromatographie b), pour fournir un perméat et un rétentat ;
- ce perméat est soumis à l'étape de concentration c) ; et
- au moins une partie des eaux mères est purgée.

Selon un sixième mode de réalisation ne faisant pas partie de la présente invention, le procédé comprend :

a) une étape de fourniture d'une composition comprenant du D-fructose ;
b) une étape d'épimérisation pour former une composition comprenant du D-fructose et du allulose ;
c) une étape de chromatographie de cette composition pour fournir une composition riche en allulose et un raffinat consistant en une composition riche en D-fructose ;
d) une étape de concentration de la composition riche en D-allulose pour former la solution mère à cristalliser ;

caractérisé en ce que :

- au moins une étape de recyclage consiste en une étape de recyclage du raffinat ;
- l'étape de nanofiltration est réalisée sur ce raffinat pour fournir un perméat et un rétentat ;
- le perméat est mélangé à la composition comprenant du D-fructose de l'étape a) ;
- l'étape d'épimérisation b) est réalisée sur ce mélange et ;
- au moins une partie du raffinat est purgée.

Selon un septième mode de réalisation ne faisant pas partie de la présente invention, le procédé comprend :

a) une étape de fourniture d'une composition comprenant du D-fructose ;
b) une étape d'épimérisation pour former une composition comprenant du D-fructose et du D-allulose ;
c) une étape de chromatographie de cette composition pour fournir une composition riche en allulose et un raffinat consistant en une composition riche en D-fructose ;
d) une étape de concentration pour former la solution mère à cristalliser ;

caractérisé en ce que :

- une étape de recyclage consiste en une étape de recyclage du raffinat obtenu à l'étape c) ;
- une première étape de nanofiltration est réalisée sur ce raffinat pour fournir un premier perméat et un premier rétentat ;
- le premier perméat est mélangé à la composition comprenant du D-fructose de l'étape a) ;
- l'étape d'épimérisation b) est réalisée sur ce mélange ;
- une étape de recyclage consiste en une étape de recyclage d'au moins une partie des eaux mères ;
- une seconde étape de nanofiltration est réalisée sur ces eaux mères recyclées pour former un second perméat et un second rétentat ;
- le second perméat est mélangé avec la composition riche en D-allulose fournie à l'étape c) ;
- ce mélange est soumis à l'étape de concentration d) pour fournir la solution mère à cristalliser et ;
- au moins une partie du raffinat et/ou des eaux mères est purgée.

Selon un huitième mode de réalisation, le procédé comprend :

a) une étape de fourniture d'une composition comprenant du D-fructose ;
b) une étape d'épimérisation pour former une composition comprenant du D-fructose et du allulose ;
c) une étape de chromatographie de cette composition pour fournir une composition riche en allulose et un raffinat consistant en une composition riche en D-fructose ;
d) une étape de concentration pour former la solution mère à cristalliser ;

caractérisé en ce que :

- une étape de recyclage consiste en une étape de recyclage du raffinat obtenu à l'étape c) ;
- une première étape de nanofiltration est réalisée sur ce raffinat pour fournir un premier perméat et un premier rétentat ;
- le premier perméat est mélangé à la composition comprenant du D-fructose de l'étape a) ;

- l'étape d'épimérisation b) est réalisée sur ce mélange ;
- une étape de recyclage consiste en une étape de recyclage d'au moins une partie des eaux mères ;
- ces eaux mères recyclées sont mélangées avec la composition riche en D-allulose fournie à l'étape c) ;
- une seconde étape de nanofiltration est réalisée sur ce mélange pour fournir un second perméat et un second rétentat ;
- ce second perméat est soumis à l'étape de concentration d) pour fournir la solution mère à cristalliser et ;
- au moins une partie du raffinat et/ou des eaux mères est purgée.

Selon un neuvième mode de réalisation ne faisant par partie de la présente invention, le procédé comprend :

a) une étape de fourniture d'une composition comprenant du D-fructose ;
b) une étape d'épimérisation pour former une composition comprenant du D-fructose et du D-allulose ;
c) une étape de chromatographie pour fournir une composition riche en D-allulose et un raffinat consistant en une composition riche en D-fructose ;
d) une étape de concentration de la composition riche en D-allulose pour former la solution mère à cristalliser ;

caractérisé en ce que :

- une étape de recyclage consiste en une étape de recyclage du raffinat obtenu à l'étape c) ;
- une première étape de nanofiltration est réalisée sur ce raffinat pour fournir un premier perméat et un premier rétentat ;
- le premier perméat est mélangé à la composition comprenant du D-fructose de l'étape a) ;
- l'étape d'épimérisation b) est réalisée sur ce mélange ;
- une étape de recyclage consiste en une étape de recyclage d'au moins une partie des eaux mères ;
- une seconde étape de nanofiltration est réalisée sur ces eaux mères recyclées pour former un second perméat et un second rétentat ;
- le second perméat est mélangé avec la composition formée à l'étape b) ;
- ce mélange est soumis à l'étape c) de chromatographie ; et
- au moins une partie du raffinat et/ou des eaux mères est purgée.

Selon un dixième mode de réalisation ne faisant par partie de la présente invention, le procédé comprend :

a) une étape de fourniture d'une composition comprenant du D-fructose ;
b) une étape d'épimérisation pour former une composition comprenant du D-fructose et du D-allulose ;
c) une étape de chromatographie pour fournir une composition riche en D-allulose et un raffinat consistant en une composition riche en D-fructose ;
d) une étape de concentration de la composition riche en D-allulose pour former la solution mère à cristalliser ;

caractérisé en ce que :

- une étape de recyclage consiste en une étape de recyclage du raffinat obtenu à l'étape c) ;
- une première étape de nanofiltration est réalisée sur ce raffinat pour fournir un premier perméat et un premier rétentat ;
- le premier perméat est mélangé à la composition comprenant du D-fructose de l'étape a) ;
- l'étape d'épimérisation b) est réalisée sur ce mélange ;
- une étape de recyclage consiste en une étape de recyclage d'au moins une partie des eaux mères ;
- ces eaux mères recyclées sont mélangées avec la composition formée à l'étape b) ;
- une seconde étape de nanofiltration est réalisée sur ce mélange pour fournir un second perméat et un second rétentat ;
- ce second perméat est soumis à l'étape de chromatographie c) ; et
- au moins une partie du raffinat et/ou des eaux mères est purgée.

Selon un onzième mode de réalisation, le procédé comprend :

a) une étape de fourniture d'une composition comprenant du D-fructose ;
b) une étape d'épimérisation pour former une composition comprenant du D-fructose et du allulose ;
c) une étape de chromatographie pour fournir une composition riche en D-allulose et un raffinat consistant en une composition riche en D-fructose ;
d) une étape de concentration de la composition riche en D-allulose pour former la solution mère à cristalliser ;

caractérisé en ce que :

- une étape de recyclage consiste en une étape de recyclage du raffinat obtenu à l'étape c) ;
- une première étape de nanofiltration est réalisée sur ce raffinat pour fournir un premier perméat et un premier rétentat ;
- le premier perméat est mélangé à la composition comprenant du D-fructose de l'étape a) ;
- l'étape d'épimérisation b) est réalisée sur ce mélange ;
- une étape de recyclage consiste en une étape de recyclage d'au moins une partie des eaux mères ;
- ces eaux mères recyclées sont mélangées avec la composition formée à l'étape b) ;
- l'étape de chromatographie c) est réalisée sur ce mélange ;
- une seconde étape de nanofiltration est réalisée sur la composition riche en D-allulose résultant de cette étape de chromatographie b), pour fournir un second perméat et un second rétentat ;
- ce second perméat est soumis à l'étape de concentration d) ; et
- au moins une partie du raffinat et/ou des eaux mères est purgée.

De manière non limitative, l'invention va maintenant être détaillée en vue d'illustrer son intérêt dans les exemples ci-après.

## **Exemples**

*Méthodes analytiques*

Chromatographie en phase gazeuse

Le chromatographe en phase gazeuse utilisé est de type Varian 3800 et est équipé de :

- Un injecteur split-splitless (avec ou sans diviseurs);

- Un détecteur à ionisation de flamme (FID) ;

- Un système informatique permettant de traiter le signal du détecteur ;

- Un échantillonneur automatique (type 8400).

Les différentes quantités sont déterminées par chromatographie en phase gazeuse sous forme de dérivés méthoximés triméthylsilylés, puis quantifiés par la méthode de l'étalonnage interne.

*Détermination des teneurs en D-allulose, D-fructose et glucose*

Les coefficients de réponse appliqués sont de 1,25 pour le D-allulose et le D-fructose et de 1,23 pour le glucose. Les autres monosaccharides n'ont pas été détectés.

*Préparation de l'échantillon*

Dans une boîte à tare, peser 100 à 300 mg de l'échantillon à tester + 10 ml solution étalon interne constituée de méthyl $\alpha$-D-glucopyranoside à 0,3mg/ml dans la pyridine. Dans un godet de 2 ml, prélever 0,5 ml de la boîte à tare et évaporer à sec sous courant d'azote. Ajouter 20 mg de chlorhydrate de méthoxylamine et 1 ml de pyridine. Boucher et laisser dans le système d'incubation de type Reacti-therm ® à 70°C pendant 40 min. Ajouter 0,5 ml de N, O Bis (triméthylsilyl) trifluoroacétamide (BSTFA). Chauffer 30 min à 70°C.

*Conditions chromatographiques*

Colonne : DB1 capillaire 40 mètres, diamètre interne 0,18 mm, épaisseur du film 0,4$\mu$m, constituée à 100 % de diméthylpolysiloxane, apolaire (J&W Scientific réf. : 121-1043)

Température de colonne : 100°C programmation jusqu'à 260°C à raison de 3°C/min, puis jusqu'à 300°C à 15°C/min, maintenir 5 min à 300°C.

Température injecteur : 300°C

Température détecteur : 300°C (Range 10$^{-12}$)

Pression : 40 psi (débit constant)

Gaz vecteur : Hélium

Mode d'injection : Split (Débit de split : 100 ml/min)

Volume injecté : 1,0 µl

Le D-allulose, le D-fructose et le glucose ont été détectés dans cet ordre. Le D-allulose, qui était inconnu, a un temps de rétention dans ces conditions compris entre 39,5 et 40 minutes.

_Détermination des teneurs en dimères de D-allulose et en dimères glucose-glucose_

Les coefficients de réponse appliqués sont de 1,15 pour les dimères de D-allulose et le maltose, et de 1,08 pour l'isomaltose. Les autres dimères de glucose n'ont pas été détectés.

_Préparation de l'échantillon :_

Dans une boîte à tare, peser 100 à 300 mg de l'échantilllon à tester + 10 ml solution étalon interne constituée de Phényl beta-D -glucopyranoside à 0,3 mg/ml dans la pyridine.

Dans un godet de 2 ml, prélever 0,5 ml de la boîte à tare et évaporer à sec sous courant d'azote. Reprendre par 0,5 ml de la solution de chlorhydrate d'hydroxylamine à 40g/l dans la pyridine, boucher, agiter et laisser 40 min à 70°C.

Ajouter 0,4 ml de BSTFA et 0,1 ml de N-Trimethylsilylimidazole (TSIM). Chauffer 30 min à 70°C.

_Conditions chromatographiques_

Colonne : DB1 capillaire 30 mètres, diamètre interne 0,32 mm, épaisseur du film 0,25 µm (J&W Scientific réf. : 123-1032)

Température de colonne : 200°C programmation jusqu'à 280°C à raison de 5°C/min (maintenir 6 min), puis jusqu'à 320°C à 5°C/min, maintenir 5 min à 320°C.

Température injecteur : 300°C

Température détecteur : 300°C (Range 10$^{-12}$)

Pression : 14 psi (débit constant)

Gaz vecteur : Hélium

Mode d'injection : Split (Débit de split : 80 ml/min)

Volume injecté : 1,2 µl

_Expression des résultats :_

La teneur des différents constituants est exprimée en g pour 100 g de produit brut et elle est donnée par l'équation suivante :

$$\% \text{ constituant } i = \frac{Si}{Se} \times \frac{Pe}{P} \times \frac{100}{Ki}$$

Avec :

Si = surface du ou des pics de constituant i

Se = surface du pic d'étalon interne

Pe = Poids d'étalon interne introduit dans le bécher (en mg)

P = poids d'échantillon pesé (en mg)

Ki = coefficient de réponse du constituant i

Si le pourcentage obtenu (exprimé ici en brut) dépasse 20% pour un des constituants, l'échantillon est dilué et l'analyse CPG recommencée afin d'obtenir une quantité massique inférieure à 20%.

Les quantités massiques exprimées en brut sont ensuite exprimées en sec, en divisant pour la matière sèche de l'échantillon testé.

Les quantités massiques de D-allulose, de D-fructose et de glucose sont déterminées aisément, aucun des pics caractéristiques n'étant co-élués.

Le pic du maltose et des dimères de D-allulose peuvent être co-élués. Il faut noter toutefois que, dans les cristaux de l'invention et décrits dans les exemples ci-après, le maltose n'est jamais présent.

Si les pics caractéristiques du maltose ne sont pas détectés, la surface Si des dimères de D-allulose est déterminée par intégration des pics inconnus, entre 10 et 17 minutes. Si les pics caractéristiques du maltose sont détectés (ce qui peut être le cas dans les sirops de l'invention), on détermine les quantités de maltose et on soustrait cette quantité à la quantité totale des dimères.

Pour déterminer la quantité totale de dimères glucose-glucose, on réalise sur un échantillon le protocole suivant :

• Hydrolyse chlorhydrique

Dans un tube à hydrolyse de 15 ml à bouchon vissable en téflon, peser environ précisément de 50 à 500 mg d'échantillon (ajuster la pesée suivant la teneur en sucre attendue), ajouter 2 ml à la pipette à deux traits de la solution d'étalon interne (galactitol à 5 mg/ml dans l'eau osmosée), ajouter 3 ml d'eau et 5 ml de la solution d'HCl 4N.

Boucher hermétiquement, agiter 1 min à l'agitateur Vortex. Placer le tube au bain à sec thermostaté régulé à 100°C pendant 1 heure en agitant au vortex de temps en temps.

• Déminéralisation et concentration

Après refroidissement, dans un bécher de 50 ml, déposer la totalité de l'hydrolyse. Ajouter 6 à 8 g d'un mélange à 50/50 de résine anionique AG4 X 4 et AG50 W 8. Laisser sous agitation magnétique pendant 5 minutes. Filtrer sur papier. Récupérer le jus et renouveler l'étape de déminéralisation jusqu'à obtention d'un pH proche de l'eau.

• Préparation de l'échantillon

Dans une boîte à tare, peser 100 à 300 mg de l'échantillon à tester + 10 ml solution étalon interne constituée de méthyl $\alpha$-D-glucopyranoside à 0,3 mg/ml dans la pyridine. Dans un godet de 2 ml, prélever 0,5 ml de la boîte à tare et évaporer à sec sous courant d'azote. Ajouter 20 mg de chlorhydrate de méthoxylamine et 1 ml de pyridine. Boucher et laisser au Reacti-therm® à 70°C pendant 40 min. Ajouter 0,5 ml de BSTFA. Chauffer 30 min à 70°C.

La quantité de glucose total de la solution (qui comprend le glucose initial dit « libre » et le glucose issu de l'hydrolyse et notamment lié à la présence de maltose et d'isomaltose) est déterminée par analyse CPG du glucose. On en déduit aisément la quantité de maltose et, par différence avec la quantité totale de dimères attribués aux pics entre 10 et 17 minutes, la quantité de dimères de D-allulose.

Granulomètre

Les valeurs de taille moyenne en volume D 4,3 ainsi que de rapport de diamètres Feret min/Feret max des cristaux sont déterminées sur un granulomètre de type QICPIC RODOS de la marque SympaTEC, équipé de son module de dispersion poudre (voie sèche), en suivant le manuel technique et les spécifications du constructeur.

***Réalisation*** *de* ***procédés*** *industriels continus de fabrication de cristaux de* ***D-allulose***

Exemple 1

L'exemple 1 consiste en une méthode de production en continu de cristaux de D-allulose. Les étapes du procédé utilisé sont détaillées dans la Figure 3. La composition et le débit des Flux après stabilisation dans le procédé sont décrits dans les Tableaux 1a et 1b.

*Etape 1 :*

On introduit dans un réacteur batch agité de 12m$^3$ utiles, 13,1 tonnes d'un mélange composé pour 26% d'un sirop de D-fructose Fructamyl (Tereos) comprenant 95% de D-fructose à 50% de matière sèche (MS) (Flux1), et 74% du Flux 16 ramené à 50%MS. Le tout (Flux 1') est maintenu à 55°C. On introduit dans la cuve un lyophilisat de la souche *Bacillus subtilis* hôte de l'enzyme D-Psicose 3 Epimerase détaillée dans le brevet WO2015032761 en quantité suffisante pour avoir 2,5*10$^7$ unités d'activité dans le réacteur. Cinq réacteurs sont utilisés séquentiellement de façon à fournir un sirop essentiellement composé de fructose et d'allulose (Flux 2) en continu à un débit de 1,3t/h. Les conditions de la réaction sont les suivantes :

- Température : 55°C

- pH=7

- Temps de réaction 48h

A l'issue de la réaction, le Flux 2 est obtenu comprend une richesse en D-allulose environ égale à 25% et une richesse en D-fructose environ égale à 75%.

*Etape 2 :*

Le Flux 2 passe à travers une membrane de microfiltration au cours d'une opération batch. On obtient un Flux 3 exempt de débris cellulaires et un rétentat de microfiltration (Flux 17) comprenant les débris issus du lyophilisat de *Bacillus subtilis* qui est purgé du circuit. Les paramètres de microfiltration sont comme suit :

- Pression transmembranaire : 0-3 bar

- Taille de pore : 0,1 $\mu$m

- Température : 50°C

- Débit moyen : 15 l/h/m$^2$

- Membrane : Sepro PS35

- Facteur Concentration Volumique : 33

Tableau 1a : Débits et composition des Flux des étapes 1 à 5 de l'Exemple 1

| Etape/Caractéristiques des Flux | Flux | | |
|---|---|---|---|
| Etape 1 | Flux 1 | Flux 16 | Flux 1' |
| Débit massique (kg/h) | 360 | 1005 | 1365 |
| Matière Sèche (%) | 50 | 50 | 50 |
| Richesse Fructose (%) | 94,5 | 75,5 | 80,5 |
| Richesse Dextrose (%) | 2 | 6,6 | 5,4 |
| Richesse Allulose (%) | 1 | 2,1 | 1,7 |
| Richesse Di-Allulose (%) | 1 | 10,9 | 8,3 |
| Richesse Autre (%) | 1,5 | 4,9 | 4,1 |
| | | | |
| Etape 2/Etape 3 | Flux2/3/4 | Flux 17 | - |
| Débit massique (kg/h) | 1324 | 41 | - |

(suite)

| Etape 2/Etape 3 | Flux2/3/4 | Flux 17 | - |
|---|---|---|---|
| Matière Sèche (%) | 50 | 50 | - |

| Etape 4 | Flux 4' | Flux 14 | Flux 5 |
|---|---|---|---|
| Débit massique (kg/h) | 1673 | 2061 | 2993 |
| Matière Sèche (%) | 50,2 | 26,2 | 10 |
| Richesse Fructose (%) | 49,6 | 75,5 | 2,8 |
| Richesse Dextrose (%) | 4,5 | 6,6 | 0,6 |
| Richesse Allulose (%) | 33,4 | 2,1 | 90,1 |
| Richesse Di-Allulose (%) | 8,3 | 10,9 | 3,5 |
| Richesse Autre (%) | 4,2 | 4,9 | 3 |

| Etape 5 | Flux 5 | Flux 6 | Flux12 |
|---|---|---|---|
| Débit massique (kg/h) | 2993 | 2797 | 196 |
| Matière Sèche (%) | 10 | 8,7 | 29 |
| Richesse Fructose (%) | 2,8 | 2,7 | 3 |
| Richesse Dextrose (%) | 0,6 | 0,6 | 0,7 |
| Richesse Allulose (%) | 90,1 | 94,4 | 71,7 |
| Richesse Di-Allulose (%) | 3,5 | 0,4 | 16,6 |
| Richesse Autre (%) | 3 | 1,9 | 8 |

Tableau 1b : Débits et composition des Flux des étapes 6 à 9 de l'Exemple 1

| Etape/Caractéristique | Flux | | |
|---|---|---|---|
| Etape 6 | Flux 6 | Flux 7 | - |
| Débit massique (kg/h) | 2797 | 279 | - |
| Matière Sèche (%) | 8,7 | 87 | - |
| Richesse Fructose (%) | 2,7 | 2,7 | - |
| Richesse Dextrose (%) | 0,6 | 0,6 | - |
| Richesse Allulose (%) | 94,4 | 93,7 | - |
| Richesse Di-Allulose (%) | 0,4 | 1,1 | - |
| Richesse Autre (%) | 1,9 | 1,9 | - |

| Etape 7a/7b | Flux7/8/9 | | - |
|---|---|---|---|
| Débit massique (kg/h) | 279 | - | - |
| Matière Sèche (%) | 87 | - | - |

| Etape 8 | Flux 9 | Flux10 | Flux 13 |
|---|---|---|---|
| Débit massique (kg/h) | 279 | 126 | 152 |
| Matière Sèche (%) | 87 | 97 | 76,5 |
| Richesse Fructose (%) | 2,7 | 0,1 | 5,4 |

(suite)

| Etape 8 | Flux 9 | Flux10 | Flux 13 |
|---|---|---|---|
| Richesse Dextrose (%) | 0,6 | 0 | 1,2 |
| Richesse Allulose (%) | 93,7 | 99,7 | 87,5 |
| Richesse Di-Allulose (%) | 1,1 | 0,2 | 2,2 |
| Richesse Autre (%) | 1,9 | 0 | 3,7 |
|  |  |  |  |
| Etape 9 | Flux 10 | Flux11 | - |
| Débit massique (kg/h) | 126 | 122 | - |
| Matière Sèche (%) | 97 | 99,8 | - |

*Etape 3 :*

Le Flux 3 est déminéralisé par passage sur une résine cationique forte Dowex 88 puis une résine anionique faible Dowex 66 à un débit moyen de 2BV/h. Les bonbonnes sont maintenues à une température de 45°C et la résistivité du Flux 4 à l'issue de la déminéralisation reste supérieure à $100 k\Omega.cm^{-1}$ en sortie (Flux 4). Dans le cas contraire la régénération des résines est opérée.

*Etape 4 :*

Le Flux 4 est mélangé au Flux 12 (rétentat de nanofiltration) et au Flux 13 (eaux mères de cristallisation) pour former un Flux 4' qui alimente la chromatographie continue à lit mobile simulé (SMB) (SCC ARI® équipé de 8 colonnes) du circuit. Le débit moyen d'alimentation est de 1673kg/h à 50%MS.
Les paramètres de la chromatographie sont définis comme suit :

- Volume/colonne : $2m^3$

- Résine : Dowex Monosphere 99Ca/320

- Température : 60°C

- Débit eau/Flux 4' (vol./vol.) : 2,4

- Load (Débit alimentation/volume résine) : $0,09\ h^{-1}$

Deux fractions sont extraites : le raffinat de la SCC (Flux 14), la fraction riche en D-allulose (Flux 5) qui part en direction de l'étape 5. 7% du Flux 14 est purgé (Flux 15) afin d'évacuer les dimères d'allulose tandis que le reste (Flux 16) repart à l'étape 1 après avoir été ramené à une matière sèche de 50% par l'intermédiaire d'un évaporateur.

*Etape 5 :*

Le Flux 5 est traité sur une installation de nanofiltration en mode batch. Les paramètres sont comme suit :

- Pression transmembranaire : 30 bars

- Température : 20°C

- Membrane : GE Duracon NF1 8040C35

- Facteur Concentration Volumique (FCV) : 16

Les dimères d'allulose se concentrent dans le rétentat (Flux 12) et ce rétentat est recyclé et mélangé avec le Flux 4, tandis que le perméat (Flux 6) est récupéré. La figure 6 donne le détail de la perméation des sirops en fonction du FCV.

*Etape 6 :*

Le Flux 6 passe à travers un évaporateur deux étages dont la pression à l'intérieur est de 50 mbar. En sortie du premier étage, le Flux est à une température de 38°C et a une matière sèche de 35%. En sortie du deuxième étage, le Flux atteint une température de 48°C et présente 87% de matière sèche. On obtient la solution-mère de D-allulose (Flux 7) à l'issue de cette étape.

*Etape 7a :*

La solution-mère ainsi formée (Flux 7) est introduite immédiatement après avoir été réchauffée à 68°C par un échangeur, dans un cristallisoir-évaporateur adiabatique sous vide de 3m$^3$ utiles, à l'intérieur duquel la pression est maintenue à 35mbars.

Le principe de fonctionnement du cristallisoir-évaporateur adiabatique sous vide utilisé ici est détaillé à la Figure 4 :

- Le Flux 7a est composé de sirop de D-allulose sursaturé à 35°C et des particules les plus fines de D-allulose. Il est mélangé au Flux 7 dans un ratio tel que le mélange se retrouve juste en dessous de la limite de solubilité du mélange (84% de matière sèche et 46°C) (Flux 7 b). Les particules fines sont ainsi refondues.

- La masse-cuite est soutirée du cristallisoir-évaporateur adiabatique à une température de 35°C, à la même vitesse que le mélange, de façon à maintenir le niveau constant dans le cristallisoir-évaporateur adiabatique. Cette masse-cuite récupérée est séparée en deux flux : le Flux 7d et le Flux 8. Le Flux 7d est entraîné avec le Flux 7b pour former un Flux 7c comprenant la solution-mère de D-allulose ainsi que des amorces pour la cristallisation. Le ratio de mélange est fait de façon à se retrouver à nouveau au-delà de la solubilité (85,5% de matière sèche et 42,7°C) afin que les cristaux puissent continuer leurs grossissements.

- Le Flux 7c est ainsi introduit dans le cristallisoir-évaporateur adiabatique sous vide pour prolonger la cristallisation et former la masse-cuite. L'eau condensée lors de ce stade est réinjectée en continu le long des parois en haut du cristallisoir.

Les différents Flux lors du stade d'évapo-refroidissement dans le cristallisoir-évaporateur adiabatique sous vide est reporté dans le Tableau 2.

Tableau 2 : Caractéristiques des différents Flux dans le cristallisoir-évaporateur adiabatique sous vide

| Paramètre | Flux 7 | Flux 7a) | Flux 7b) | Flux 7c) | Flux 7d) | Flux 8 |
|---|---|---|---|---|---|---|
| Débit massique (kg/h) | 279 | 519 | 798 | 1203 | 405 | 279 |
| Matière Sèche (%) | 87 | 83 | 84,1 | 85,1 | 87 | 87 |
| Température (°C) | 68,3 | 35 | 46 | 42,4 | 35 | 35 |

*Etape 7b :*

La masse-cuite soutirée est injectée en tête de cristallisoir vertical de volume utile de 8m$^3$ et équipé d'un agitateur ainsi que de cinq nappes de refroidissement. La masse-cuite est amenée de 35 à 20°C en 40h, soit une rampe de refroidissement d'environ 0,4°C/h. Le schéma du cristallisoir est présenté dans la Figure 5. La température de l'eau de refroidissement dans les nappes est la suivante :

1. 34°C en entrée, 32°C en sortie

2. 31°C en entrée, 29°C en sortie

3. 28°C en entrée, 26°C en sortie

4. 25°C en entrée, 22°C en sortie

5. 21°C en entrée, 20°C en sortie

*Etape 8 :*

En bas de cristallisoir, la suspension de cristaux (Flux 9) est récupérée puis centrifugée sur une essoreuse Rousselet Robatel SC 100KSA. La suspension de cristaux est systématiquement centrifugeable, ce qui signifie que le procédé est très stable au cours du temps. Les eaux-mères (Flux 13) sont recyclées et mélangées aux Flux 4 et 12. On récupère les cristaux humides de D-allulose (Flux 10). Un premier clairçage à l'eau puis un clairçage final à l'éthanol de l'ordre de 0,5% m/m est réalisé pour améliorer la séparation. Les cristaux clairçés comprennent 3% d'eau.

*Etape 9 :*

Les cristaux humides passent à travers un séchoir rotatif et on obtient des cristaux séchés qui comprennent 0,4% d'eau. Un refroidissement final dans un lit fluidisé abaisse la température des cristaux de 60 à 25°C. Les cristaux finaux sont récupérés (Flux 11) puis conditionnés. Le rendement global en cristaux de D-allulose, qui est le rapport exprimé en masse sèche de la masse de cristaux de D-allulose obtenue sur la masse de D-fructose introduite, est de 72%.

Exemple 2

L'Exemple 2 est identique à l'Exemple 1 à la différence qu'aucun recyclage n'est réalisé. Le Flux 7 comprend 0,7% de dimères de D-allulose. La suspension de cristaux du Flux 9 est toujours centrifugeable au cours du temps. Bien que le rendement global en cristaux soit de 12% uniquement, le procédé présente l'avantage d'être stable, contrairement aux procédés des exemples comparatifs, n'utilisant pas l'étape de nanofiltration, qui vont être présentés ci-après.

Exemple comparatif 1

L'Exemple comparatif 1 est identique à l'Exemple 1 à la différence :

- qu'aucune étape de nanofiltration n'est réalisée,

- qu'aucune étape de recyclage des eaux mères n'est effectuée,

- que le recyclage du raffinat (Flux 14) est recyclé en intégralité pour être mélangé avec le Flux 1 du sirop de D-fructose,

- l'étape de cristallisation est réalisée comme suit : la solution-mère formée (Flux 7) est introduite séquentiellement dans trois cristallisoirs verticaux de volume utile de 8m$^3$ identique à celui utilisé pour l'étape 7b de cristallisation de l'exemple 1. La rampe de refroidissement est de 0,33°C/h jusque 20°C. Dans chaque cristallisoir, on introduit une amorce de D-allulose de D4,3 environ égal à 60$\mu$m dans des quantités massiques de 0,1%, cette quantité étant exprimée par rapport au poids sec de D-allulose introduit dans le cristallisoir.

Le circuit de production utilisé *(i.e* les différentes étapes du procédé utilisé) est celui correspondant à la Figure 1.

Le Flux 7 comprend 1,9% de dimères de D-allulose. On récupère un Flux 9 qui est conduit à l'étape 8. Ce Flux 9 est une masse-cuite qui n'est pas toujours centrifugeable. Au bout d'une semaine, le Flux 7 comprend 2,2% de dimères de D-allulose et la masse-cuite du Flux 9 devient même systématiquement non centrifugeable (cristaux de taille trop petite).

Exemple comparatif 2

L'Exemple comparatif 2 est identique à l'Exemple comparatif 1 à la différence que les eaux mères sont totalement recyclées (Flux 13) pour être mélangées avec la composition riche en D-allulose issue de l'étape de chromatographie (Flux 5) et que le raffinat (Flux 14) n'est pas recyclé et est purgé du circuit.

Le circuit de production utilisé est celui correspondant à la Figure 2.

Le Flux 7 comprend 1,9% de dimères de D-allulose. On récupère un Flux 9 qui est conduit à l'étape 8. Ce Flux 9 est une masse-cuite qui n'est pas toujours centrifugeable. Dès le recyclage des eaux mères, le Flux 7 comprend 2,4% de dimères de D-allulose et la masse-cuite du Flux 9 devient même systématiquement non centrifugeable (cristaux de taille trop petite).

Exemple comparatif 3

L'Exemple comparatif 3 est identique à l'Exemple comparatif 1 à la différence que le raffinat n'est pas recyclé.

Le circuit de production utilisé est celui correspondant à la Figure 1.

Le Flux 7 comprend 1,9% de dimères de D-allulose. On récupère un Flux 9 qui est conduit à l'étape 8. Ce Flux 9 est une masse-cuite qui n'est pas toujours centrifugeable. Lorsqu'elle est centrifugeable, les eaux-mères se séparent des cristaux d'allulose qui peuvent être récupérés. Mais parfois, le Flux 9 consiste en un massé de petits cristaux indissociables, synonyme d'une nucléation spontanée dans le cristallisoir. Dans ce cas-là, il est nécessaire de vidanger le Flux du circuit. Ceci rend le procédé inexploitable industriellement.

Le récapitulatif des résultats obtenus pour ces procédés sont reportés dans le Tableau 3. Le rendement reporté global est une moyenne sur une semaine d'utilisation.

Les caractéristiques des cristaux obtenus pour les exemples 1, 2 et l'exemple comparatif 3, ainsi que les cristaux de D-allulose commercialisés par la société CJ Cheiljedang Food Ingredient sont reportées au Tableau 4.

Tableau 3 : Comparaison des différents circuits testés

| Exemple | Circuit | Recyclage du Flux 14 (%) | Recyclage du Flux 13 (%) | Recyclage du Flux 12 (%) | Rendement global en cristaux de D-allulose du procédé continu |
|---|---|---|---|---|---|
| Exemple comparatif 1 | Figure 1 | 100 | 0 | 0 (pas de nanofiltration) | Instable puis arrêt installation, ne fonctionne pas en continu |
| Exemple comparatif 2 | Figure 2 | 0 | 100 | 0 (Pas de nanofiltration) | Instable puis arrêt installation, ne fonctionne pas en continu |
| Exemple comparatif 3 | Figure 1 | 0 | 0 | 0 (pas de nanofiltration) | Instable, ne fonctionne pas en continu |
| Exemple 1 | Figure 3 | 93 | 100 | 100 | Stable, 72% |
| Exemple 2 | Figure 3 | 0 | 0 | 0 | Stable, 12% |

Le procédé de l'invention permet d'obtenir une cristallisation stable au cours du temps, ce qui est démontré dans le procédé industriel exemplifié ci-dessus (Exemples 1 et 2). Il permet en outre de réaliser des recyclages très importants et d'augmenter ainsi le rendement global de cristaux en D-allulose (voir Exemple 2). En effectuant des recyclages sans s'assurer de séparer les dimères de D-allulose par l'étape de nanofiltration, les exemples comparatifs illustratifs 1 et 2 ci-dessus ont démontré que le procédé de cristallisation industriel devait être arrêté car la masse-cuite devient systématiquement non centrifugeable (cristaux de taille trop petite).

Tableau 4: Caractéristique des cristaux obtenus

| Cristaux | Humidité résiduelle | D4,3 (μm) | % dimères de D-allulose (CPG) | Feret min/Feret max à 200 μm | Feret min/Feret max à 400 μm |
|---|---|---|---|---|---|
| Exemple 1 | 0,3% | 302 | 0,2% | 0,63 | 0,68 |
| Exemple 2 | 0,3% | 285 | <0,1% | 0,68 | 0,76 |
| Exemple comparatif 3 | 0,3% | 297 | 0,7% | 0,55 | 0,53 |
| Cristaux commercialisés par CJ Cheiljedang Food Ingredient | 0,5% | 346 | 0,7% | 0,53 | 0,50 |

La Figure 14 qui représente, pour les cristaux de l'Exemple 2 et les cristaux commercialisés par CJ de D-allulose, le rapport des diamètres Feret min/Feret max en fonction de la tailles de particules en volume D4,3, démontre que c'est pour les populations de taille importante, supérieure ou égale à 200 μm (par exemple dans la gamme allant de 200 à 400 μm), que de nettes différences d'aspect sont observées entre les cristaux selon l'invention et les cristaux comparatifs. Ainsi, dans cette gamme allant de 200 à 400 μm, les rapports Feret min/Feret max sont, pour les cristaux comparatifs, toujours inférieurs à 0,55 alors que les cristaux selon l'invention présentent un rapport d'au moins 0,63. Cette différence est tout à fait en accord avec les images de microscopie optique des Figures 11 et 12, qui démontrent visuellement que les cristaux selon l'invention présentent un aspect bien plus trapu que les cristaux comparatifs. En ce qui concerne les cristaux de CJ de la Figure 13 (images de microscopie optique), on remarque qu'il s'agit de cristaux sous forme d'aiguilles, non individualisés.

Bien que l'exemple comparatif 3 ne soit pas strictement identique à l'enseignement du document WO 2011119004 A2 en ce qui concerne l'étape de concentration (notamment en ce que la Demanderesse est parvenue à réaliser de manière plus optimale l'étape de concentration de manière à réduire encore la quantité de dimères de D-allulose formée), les cristaux obtenus utilisent un procédé de même type que celui utilisé pour fabriquer les cristaux décrits à l'exemple 6 du document WO 2011119004 A2 (notamment en ce qui concerne la cristallisation se fait exclusivement par refroidissement contrôlé dans l'eau). Cet essai comparatif 3, produisant des cristaux comprenant 0,7% de dimères de D-allulose, démontre donc bien que le document WO 2011119004 A2 ne permet pas de former les cristaux de l'invention.

***Utilisation des cristaux de l'invention dans différentes applications***

Les cristaux de l'Exemple 2 ont été utilisés dans la fabrication de produits qui suivent.

Fabrication de boissons de substitut de repas

L'objectif est de créer une boisson en poudre de substitut de repas comprenant peu de calories. Cette boisson en poudre doit pouvoir présenter un bon écoulement et former peu de grumeaux lorsqu'elle est formulée.

**Formule** :

| Ingrédients | Pourcentages | |
|---|---|---|
| | Allulose | Sucrose |
| Sucre | 28,29 | 28,29 |
| Concentré de protéïnes de lait | 26,62 | 26,62 |
| GLUCIDEX® 19 maltodextrine | 22,63 | 22,63 |
| NUTRIOSE® FM 06 Fibre soluble | 10,82 | 10,82 |
| Protéine de pois | 9,98 | 9,98 |
| Caséïnate de sodium | 0,67 | 0,67 |
| Arome crème de vanille | 0,50 | 0,50 |
| Arome vanille | 0,33 | 0,33 |
| Cekol 10000 | 0,17 | 0,17 |
| Total | 100,00 | 100,00 |

Après avoir été pesés, les ingrédients sont vigoureusement mélangés dans un mélangeur à sec.
La boisson est ensuite aisément reconstituée en ajoutant 210 g d'eau à 30 g de la formule, sans former de grumeaux.
La formule utilisant les cristaux de l'invention présentent un comportement à l'écoulement tout à fait similaire à la formule comprenant du sucrose, tout en comprenant bien moins de calories.

Fabrication de gâteau jaune

L'objectif est de fournir un gâteau jaune présentant une texture et apparence satisfaisantes dont la teneur calorique est réduite de 25%.

**Formule** :

| Ingrédients | Référence | Invention |
|---|---|---|
| Sucrose | 24,10 | 0,00 |
| Farine à gâteau | 27,09 | 25,24 |

(suite)

| Ingrédients | Référence | Invention |
|---|---|---|
| Nutriose® FB06 | 0,0 | 1,75 |
| Jaune d'oeuf | 10,00 | 10,00 |
| Beurre | 15,45 | 15,45 |
| Lait | 21,81 | 21,81 |
| Sel | 0,25 | 0,25 |
| Levure chimique | 0,80 | 0,90 |
| Vanille | 0,50 | 0,50 |
| Allulose | 0,00 | 24,10 |
| Total | 100,00 | 100,00 |

**Méthode** :

1. Mélanger la farine, le sel, le Nutriose® et la levure ;

2. Crémer le beurre avec le sucrose ou l'allulose ;

3. Ajouter le jaune d'œuf et la vanille à la crème puis ajouter le lait pour former un mélange crémeux ;

4. Ajouter le mélange comprenant la farine dans le mélange crémeux et mélanger dans un mixeur en position lente (1 minute) puis plus vivement jusqu'à ce que la formule soit bien mélangée ;

5. Verser 600 g de la pâte dans un moule circulaire graissé de 9 pouces ;

6. Cuire à 180°C pendant 20 minutes.

L'objectif est atteint : le gâteau utilisant les cristaux de l'invention présente une texture très agréable en bouche (on parle de « crumb texture ») et la forme du gâteau se maintient après cuisson.

Fabrication de cookies au chocolat

Les formules suivantes ont été réalisées :

| Ingrédients | Référence | Allulose | Allulose + Nutriose® |
|---|---|---|---|
| Farine de blé | 25 | 25 | 23 |
| Bicarbonate de soude | 0,14 | 0,14 | 0,14 |
| Sel | 0,17 | 0,17 | 0,17 |
| Beurre fondu | 14,16 | 14,16 | 14,16 |
| Nutriose® FB06 | 0 | 0 | 2 |
| Allulose | 0 | 29,17 | 29,17 |
|  |  |  |  |
| Sucre brun | 20,35 | 0 | 0 |
| Sucre en poudre | 8,82 | 0 | 0 |
| Vanille | 1,14 | 1,14 | 1,14 |
| Oeufs | 7 | 7 | 7 |

(suite)

| Ingrédients | Référence | Allulose | Allulose + Nutriose® |
|---|---|---|---|
| Pépites de chocolat | 23,22 | 23,22 | 23,22 |
| Total | 100 | 100 | 100 |

**Méthode** :

1. Mélanger les ingrédients secs entre eux;

2. Crémer le beurre avec les sucres ou l'allulose ;

3. Ajouter les œufs et la vanille au mélange crémeux ;

4. Ajouter le mélange comprenant la farine dans le mélange crémeux et mélanger dans un mixeur en position lente (1 minute) puis plus vivement jusqu'à ce que la formule soit bien mélangée ;

5. Ajouter les pépites de chocolat et mélanger ;

6. Peser des portions de 30 g and faire cuire à 160°C pendant 8 minutes.

La pâte à cookies à base d'allulose (colonne 2) s'étale moins que la pâte à base de sucres (colonne 1). Toutefois, la pâte de la colonne 3 s'étale de la même manière que la pâte de la colonne 1.
Pendant la cuisson, les cookies à base d'allulose brunissent plus rapidement.
Il faut noter que les cookies présentent une forme de dôme. La hauteur du cookie selon l'invention est moins gonflée et celui-ci ne s'effondre pas après cuisson, contrairement au cookie à base de sucres, ce qui lui permet de garder un meilleur aspect.
Les cookies à base d'allulose présentent un bon goût, bien que moins sucré. La texture des cookies selon l'invention est tendre et plus humide que les cookies à base de sucres.
L'activité de l'eau (w a) et l'humidité (M) des cookies sont mesurés au cours du temps :

| Date | Référence | | Allulose | | Allulose + Nutriose® | |
|---|---|---|---|---|---|---|
| | w a | Humidité (%) | w a | Humidité (%) | w a | Humidité (%) |
| Jour 1 | 0,6429 | 7,62 | 0,5482 | 8,91 | 0,5027 | 8,69 |
| Jour 7 | 0,7531 | 9,42 | 0,5859 | 9,59 | 0,5472 | 8,82 |
| Jour 30 | 0,7633 | 8,33 | 0,5888 | 9,47 | 0,5563 | 9,11 |

Les cookies à base d'allulose présentent une meilleure stabilité à l'humidité.

Fabrication de cookies aux flocons d'avoine

**Formules** :

| Ingrédients | Référence | Invention |
|---|---|---|
| Farine de blé | 16,84 | 15,09 |
| Nutriose® FB06 | 0,00 | 1,75 |
| Bicarbonate de soude | 0,42 | 0,42 |
| Levure | 0,27 | 0,27 |

(suite)

| Ingrédients | Référence | Invention |
|---|---|---|
| Sel | 0,44 | 0,44 |
| Beurre | 14,53 | 14,53 |
| Sucrose | 14,08 | 11,30 |
| Sucre roux | 13,89 | 0,00 |
| Allulose | 0,00 | 16,67 |
| Oeufs | 6,43 | 6,43 |
| Vanille | 0,60 | 0,60 |
| Flocons d'avoine | 19,35 | 19,35 |
| Inclusions* | 13,15 | 13,15 |
| Total | 100,00 | 100,00 |

*Les inclusions comprennent 50,0 grammes de noix de pécan, 20,0 grammes de canneberges (cranberries) et 18,6 grammes de myrtilles.

**Méthode:**

1. Mélanger la farine, le Nutriose®, le bicarbonate de soude, la levure et le sel;

2. Crémer le beurre avec les sucres ou l'allulose ;

3. Ajouter les oeufs et la vanilla au mélange crémeux ;

4. Ajouter le mélange comprenant la farine dans le mélange crémeux et mélanger dans un mixeur en position lente (1 minute) puis plus vivement jusqu'à ce que la formule soit bien mélangée ;

5. Ajouter les flocons d'avoine et mélanger ;

6. Ajouter les inclusions et mélanger ;

7. Peser des portions de 30 g et faire cuire à 160°C pendant 10 minutes.

Les cookies obtenus utilisant de l'allulose au lieu du sucrose, sont légèrement plus bruns et présentent une texture craquante après cuisson.

Fabrication de Bubble gum

Un bubble gum a été fabriqué avec la recette ci-dessous :

| Ingrédients | Parts |
|---|---|
| Gomme de base Flama T | 24 |
| Allulose | 50 |
| Lycasin® 85/55 | 10 |
| Nutriose® FB06 | 13,4 |
| Arôme liquide | 0,9 |
| Arôme en poudre | 1,2 |
| Acidifiant | 0,5 |

(suite)

| Ingrédients | Parts |
|---|---|
| Total | 100 |

Le bubble gum présente un aspect tout à fait satisfaisant, similaire aux bubble-gums du commerce.

**Revendications**

1. Procédé de fabrication de cristaux de D-allulose comprenant :

   • une étape de fourniture d'une composition riche en D-allulose,
   • une étape de nanofiltration de ladite composition riche en D-allulose pour fournir un rétentat et un perméat, l'étape de nanofiltration étant réalisée avec une membrane ayant un seuil de coupure inférieur à 300 Da, de préférence allant de 150 à 250 Da;
   • une étape de récupération du perméat de nanofiltration ;
   • une étape de concentration de ce perméat pour fournir la solution mère de D-allulose ;
   • une étape de cristallisation de la solution mère pour former des cristaux de D-allulose et des eaux mères.

2. Procédé selon la revendication 1, **caractérisé en ce que** la solution mère à cristalliser obtenue après concentration comprend en masse sèche :

   • de 80 à 99% de D-allulose, de préférence de 85 à 98% ;
   • de 0 à 20% de D-fructose, de préférence de 0,5 à 15% ;
   • de 0 à 10% de glucose, de préférence de 0 à 5% ;
   • de 0 à 1,5% de dimères de D-allulose, par exemple de 0,1 à 1,2%, préférentiellement de 0,4 à 1,1%,
   les quantités étant déterminées par chromatographie en phase gaz (CPG) selon la méthode décrite à la page 12 ligne 22 à la page 13 ligne 24 ainsi qu'aux pages 36-39.

3. Procédé selon l'une des revendications précédentes **caractérisé en ce que** le facteur de concentration volumique de la nanofiltration va de 5 à 20.

4. Procédé selon l'une des revendications précédentes **caractérisé en ce que** l'étape de cristallisation comprend :

   i. un stade d'évapo-refroidissement adiabatique, réalisé dans un cristallisoir-évaporateur adiabatique sous vide pour former une massecuite,
   ii. suivi d'un stade de cristallisation par refroidissement de ladite massecuite pour former des cristaux.

5. Procédé selon la revendication 4 **caractérisé en ce que** la température lors du stade d'évapo-refroidissement adiabatique va de 30 à 40°C, de préférence allant de 33 à 37°C, par exemple d'environ 35°C.

6. Procédé selon l'une des revendications précédentes **caractérisé en ce qu'**il est continu.

7. Procédé selon l'une des revendications précédentes **caractérisé en ce qu'**il comprend au moins une étape de recyclage.

8. Procédé selon l'une des revendications précédentes **caractérisé en ce qu'**il comprend une étape de recyclage d'au moins une partie des eaux mères.

9. Procédé selon l'une des revendications précédentes **caractérisé en ce qu'**il comprend une étape de recyclage d'au moins une partie du rétentat.

10. Procédé selon l'une des revendications précédentes **caractérisé en ce que** l'étape de fourniture de la composition riche en D-allulose comprend :

    • une étape de fourniture d'une composition comprenant du D-fructose ;

• une étape d'épimérisation pour former une composition comprenant du D-fructose et du D-allulose ;
• une étape de chromatographie pour fournir une composition riche en D-allulose et une composition riche en D-fructose.

**11.** Procédé selon la revendication 10 **caractérisé en ce qu'**il comprend une étape de recyclage d'au moins une partie de la composition riche en D-fructose.

**12.** Procédé selon la revendication 11 **caractérisé en ce que** le taux de recyclage de la composition riche en D-fructose va de 50 à 95%.

**13.** Cristaux de D-allulose comprenant une teneur massique en dimère de D-allulose, déterminée par chromatographie en phase gaz (CPG) selon la méthode décrite à la page 12 ligne 22 à la page 13 ligne 24 ainsi qu'aux pages 36-39, inférieure à 0,50%, et présentant une taille moyenne en volume D4,3 supérieure à 200 $\mu$m, avantageusement allant de 210 à 800 $\mu$m, préférentiellement de 220 à 350 $\mu$m, et pour une taille de particules en volume D4,3 donnée et choisie dans la gamme allant de 200 à 400 $\mu$m, un rapport Feret min/Feret max supérieur à 0,60, avantageusement allant de 0,62 à 0,90, par exemple de 0,63 à 0,80, les valeurs de taille moyenne en volume D 4,3 ainsi que de rapport Feret min/Feret max étant déterminées sur un granulomètre de type QICPIC RODOS de la marque SympaTEC, équipé de son module de dispersion poudre.

**14.** Cristaux de D-allulose selon la revendication 13 **caractérisés en ce qu'**ils comprennent une teneur massique en dimère de D-allulose allant de 0,01 à 0,48%, préférentiellement allant de 0,02 à 0,45%, par exemple allant de 0,03 à 0,40%, notamment de 0,04 à 0,30%.

**15.** Cristaux de D-allulose selon la revendication 14 **caractérisés en ce qu'**ils présentent ce rapport Feret min/Feret max sur l'ensemble des tailles de particules en volume D4,3 dans la gamme allant de 200 à 400 $\mu$m.

**16.** Utilisation d'une unité de nanofiltration dans un circuit de production de cristaux de D-allulose pour améliorer le rendement global en cristaux de D-allulose, la membrane utilisée pour la nanofiltration ayant un seuil de coupure inférieur à 300 Da, de préférence allant de 150 à 250 Da.

**Patentansprüche**

**1.** Verfahren zur Herstellung von D-Allulosekristallen, umfassend:

• einen Schritt der Bereitstellung einer D-Allulose-reichen Zusammensetzung,
• einen Schritt der Nanofiltration der D-Allulose-reichen Zusammensetzung, um
• ein Retentat und ein Permeat bereitzustellen, wobei der Nanofiltrationsschritt durchgeführt wird mit einer Membran mit einer Trennschwelle von weniger als 300 Da, bevorzugt im Bereich von 150 bis 250 Da;
• einen Schritt der Rückgewinnung des Nanofiltrationspermeats;
• einen Schritt der Konzentration dieses Permeats, um die D-Allulose-Stammlösung zu erhalten;
• einen Schritt der Kristallisation der Stammlösung, um D-Allulose-Kristalle und Mutterlaugen zu bilden.

**2.** Verfahren nach Anspruch 1, **dadurch gekennzeichnet, dass** die nach der Konzentration erhaltene zu kristallisierende Stammlösung in der Trockenmasse umfasst:

• 80 bis 99 % D-Allulose, bevorzugt 85 bis 98 %;
• 0 bis 20 % D-Fructose, bevorzugt 0,5 bis 15 %;
• 0 bis 10 % Glucose, bevorzugt 0 bis 5 %;
• 0 bis 1,5 % D-Allulose-Dimere, beispielsweise 0,1 bis 1,2 %, bevorzugt 0,4 bis 1,1 %,

wobei die Mengen durch Gaschromatographie (GC) gemäß dem auf Seite 12, Zeile 22 bis Seite 13, Zeile 24 sowie auf den Seiten 36 bis 39 beschriebenen Verfahren bestimmt werden.

**3.** Verfahren nach einem der vorhergehenden Ansprüche, **dadurch gekennzeichnet, dass** der Volumenkonzentrationsfaktor der Nanofiltration zwischen 5 und 20 liegt.

**4.** Verfahren nach einem der vorhergehenden Ansprüche, **dadurch gekennzeichnet, dass** der Kristallisationsschritt umfasst:

> i. eine adiabatische Verdampfungskühlung, die in einem adiabatischen Vakuumkristallisator durchgeführt wird, um eine Schmelze zu bilden,
> ii. gefolgt von einer Kristallisationsstufe durch Abkühlen der Schmelze, um Kristalle zu bilden.

5. Verfahren nach Anspruch 4, **dadurch gekennzeichnet, dass** die Temperatur während der adiabatischen Verdampfungskühlungsphase zwischen 30 und 40 °C, bevorzugt zwischen 33 und 37 °C, beispielsweise bei etwa 35 °C, liegt.

6. Verfahren nach einem der vorhergehenden Ansprüche, **dadurch gekennzeichnet, dass** es kontinuierlich ist.

7. Verfahren nach einem der vorhergehenden Ansprüche, **dadurch gekennzeichnet, dass** es wenigstens einen Recycling-Schritt umfasst.

8. Verfahren nach einem der vorhergehenden Ansprüche, **dadurch gekennzeichnet, dass** es einen Recycling-Schritt für wenigstens einen Teil der Mutterlaugen umfasst.

9. Verfahren nach einem der vorhergehenden Ansprüche, **dadurch gekennzeichnet, dass** es einen Schritt des Recycelns wenigstens eines Teils des Retentats umfasst.

10. Verfahren nach einem der vorhergehenden Ansprüche, **dadurch gekennzeichnet, dass** der Schritt der Bereitstellung der D-Allulose-reichen Zusammensetzung umfasst:

> • einen Schritt der Bereitstellung einer Zusammensetzung, die D-Fructose umfasst;
> • einen Epimerisierungsschritt zur Bildung einer Zusammensetzung, die D-Fructose und D-Allulose umfasst;
> • einen Chromatographieschritt zur Bereitstellung einer D-Allulose-reichen Zusammensetzung und einer D-Fructose-reichen Zusammensetzung.

11. Verfahren nach Anspruch 10, **dadurch gekennzeichnet, dass** es einen Schritt des Recycelns wenigstens eines Teils der D-Fructose-reichen Zusammensetzung umfasst.

12. Verfahren nach Anspruch 11, **dadurch gekennzeichnet, dass** der Recyclinganteil der D-Fructose-reichen Zusammensetzung zwischen 50 und 95 % liegt.

13. D-Allulose-Kristalle mit einem Massenanteil an D-Allulose-Dimeren, bestimmt durch Gaschromatographie (GC) gemäß dem auf Seite 12, Zeile 22, Seite 13, Zeile 24 sowie auf den Seiten 36-39 beschriebenen Verfahren, von weniger als 0,50 % und mit einer mittleren Volumengrößen D4,3 von mehr als 200 $\mu$m, vorteilhafterweise im Bereich von 210 bis 800 $\mu$m, bevorzugt von 220 bis 350 $\mu$m, und bei einer gegebenen Volumenpartikelgröße D4,3, und gewählt im Bereich von 200 bis 400 $\mu$m, einem Verhältnis Feret min/Feret max von mehr als 0,60, vorteilhafterweise im Bereich von 0,62 bis 0,90, beispielsweise von 0,63 bis 0,80, wobei die Werte für die mittlere Volumengröße D 4,3 sowie das Verhältnis Feret min/Feret max mit einem Partikelmessgerät vom Typ QICPIC RODOS der Marke SympaTEC, ausgestattet mit einem Pulverdispersionsmodul, bestimmt werden.

14. D-Allulosekristalle nach Anspruch 13, **dadurch gekennzeichnet, dass** sie einen Massenanteil an D-Allulosedimeren von 0,01 bis 0,48 % aufweisen, bevorzugt von 0,02 bis 0,45 %, beispielsweise zwischen 0,03 und 0,40 %, insbesondere zwischen 0,04 und 0,30 %.

15. D-Allulosekristalle nach Anspruch 14, **dadurch gekennzeichnet, dass** sie dieses Verhältnis Feret min/Feret max über alle Partikelgrößen im Volumenbereich 04,3 im Bereich von 200 bis 400 $\mu$m aufweisen.

16. Verwendung einer Nanofiltrationseinheit in einem Kreislauf zur Herstellung von D-Allulosekristallen zur Verbesserung der Gesamtausbeute an D-Allulosekristallen, wobei die zur Nanofiltration verwendete Membran eine Trennschwelle von weniger als 300 Da, bevorzugt im Bereich von 150 bis 250 Da, aufweist.

## EP 3 565 656 B1

**Claims**

1. Method for producing D-allulose crystals comprising:

   • a step of providing a composition rich in D-allulose,
   • a nanofiltration step of said composition rich in D-allulose so as to provide a retentate and a permeate, said nanofiltration step being performed with a membrane having a cut-off threshold of less than 300 Da, preferably ranging from 150 to 250 Da;
   • a step of recovering the nanofiltration permeate;
   • a step of concentrating said permeate so as to provide a D-allulose stock solution;
   • a step of crystallising the stock solution so as to form D-allulose crystals and mother liquors.

2. Method according to claim 1, **characterised in that** the stock solution to be crystallised obtained after concentration comprises by dry mass:

   • 80 to 99% D-allulose, preferably 85 to 98%;
   • 0 to 20% D-fructose, preferably 0.5 to 15%;
   • 0 to 10% glucose, preferably 0 to 5%;
   • 0 to 1.5% D-allulose dimers, for example from 0.1 to 1.2%, preferably from 0.4 to 1.1%,

   the quantities being determined by gas chromatography (GC) according to the method described on page 12 line 22 to page 13 line 24 as well as on pages 36-39.

3. Method according to any one of the preceding claims, **characterised in that** the volume concentration factor of the nanofiltration ranges from 5 to 20.

4. Method according to one of the preceding claims, **characterised in that** the crystallisation step comprises:

   i. an adiabatic evaporative cooling stage, carried out in an adiabatic crystallizer evaporator under vacuum to form a massecuite,
   ii. followed by a stage of crystallisation by cooling of said massecuite so as to form crystals.

5. Method according to claim 4, **characterised in that** the temperature during the adiabatic evaporative cooling stage ranges from 30 to 40°C, preferably ranging from 33 to 37°C, for example about 35°C.

6. Method according to any one of the preceding claims, **characterised in that** it is continuous.

7. Method according to any one of the preceding claims, **characterised in that** it comprises at least one recycling step.

8. Method according to any one of the preceding claims, **characterised in that** it comprises a step of recycling at least one part of the mother liquors.

9. Method according to any one of the preceding claims, **characterised in that** it comprises a step of recycling at least one part of the retentate.

10. Method according to any one of the preceding claims, **characterised in that** the step of providing the composition rich in D-allulose comprises:

    • a step of providing a composition comprising D-fructose;
    • an epimerization step so as to form a composition comprising D-fructose and D-allulose;
    • a chromatography step so as to provide a composition rich in D-allulose and a composition rich in D-fructose.

11. Method according to claim 10, **characterised in that** it comprises a step of recycling at least one part of the composition rich in D-fructose.

12. Method according to claim 11 **characterised in that** the recycling rate of the composition rich in D-fructose ranges from 50 to 95%.

**13.** D-allulose crystals comprising a mass content of D-allulose dimer, determined by gas chromatography (GC) according to the method described on page 12 line 22 to page 13 line 24 as well as on pages 36-39, of less than 0.50%, and having a mean volume size D4.3 greater than 200 $\mu$m, advantageously ranging from 210 to 800 $\mu$m, preferably from 220 to 350 $\mu$m, and for a given and chosen volume particle size D4.3 ranging from 200 to 400 $\mu$m, a Feret min/Feret max ratio greater than 0.60, advantageously ranging from 0.62 to 0.90, for example from 0.63 to 0.80, the values of mean volume size D 4.3 as well as of Feret min/Feret max ratio being determined on a SympaTEC QICPIC RODOS granulometer, equipped with its powder dispersion unit.

**14.** D-allulose crystals according to claim 13, **characterised in that** they comprise a mass content of D-allulose dimer ranging from 0.01 to 0.48%, preferably ranging from 0.02 to 0.45%, for example ranging from 0.03 to 0.40%, in particular from 0.04 to 0.30%.

**15.** D-allulose crystals according to claim 14, **characterised in that** they have said Feret min/Feret max ratio over all volume particle sizes D4.3 in the range from 200 to 400 $\mu$m.

**16.** Use of a nanofiltration unit in a D-allulose crystal production circuit so as to improve the overall yield in D-allulose crystals, the membrane used for nanofiltration having a cut-off threshold of less than 300 Da, preferably ranging from 150 to 250 Da.

D-Fructose

*Flux 1*

*Flux 1'*

Etape 1 : Epimérisation

*Flux 2*

*Flux 14*

Etape 2 : Microfiltration ⟶ Flux 17

*Flux 3*

Etape 3 : Déminéralisation

*Flux 4*

Etape 4 : Chromatographie

*Flux 5*

Etape 6 : Evaporation

*Flux 7*

Etape 8 : Cristallisation

*Flux 9*

Etape 9 : Centrifugation ⟶ *Flux 13*

*Flux 10*

Etape 10 : Séchage

*Flux 11*

Cristaux finaux

**Figure 1**

D-Fructose

*Flux 1*

Etape 1 : Epimérisation

*Flux 2*

Etape 2 : Microfiltration ⟶ Flux 17

*Flux 3*

Etape 3 : Déminéralisation

*Flux 4*

*Flux 14* ⟵ Etape 4 : Chromatographie

*Flux 5*

Etape 6 : Evaporation

*Flux 7*

Etape 8 : Cristallisation

*Flux 9*

*Flux 13*

Etape 9 : Centrifugation

*Flux 10*

Etape 10 : Séchage

*Flux 11*

Cristaux finaux

**Figure 2**

**Figure 3**

Figure 4

Flux 8

| | |
|---|---|
| 34°C | 34°C |
| 32°C | 32°C |
| 31°C | 31°C |
| 29°C | 29°C |
| 28°C | 28°C |
| 26°C | 26°C |
| 25°C | 25°C |
| 23°C | 23°C |
| 22°C | 22°C |
| 20°C | 20°C |

Flux 9

Figure 5

Débit l/m²/H

FCV

Facteur de Concentration Volumique

**Figure 6**

**Figure 7**

**Figure 8**

**Figure 9**

**Figure 10**

Figure 11

Figure 12

**Figure 13**

Taille de particule en µm

**Figure 14**

» Feret$_{min}$ / Feret$_{max}$

**Figure 15**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015032761 A1
- JP 2001354690 A
- WO 2015094342 A
- EP 1860195 A
- JP 4761424 B
- WO 2016012853 A
- CN 104447888 A
- WO 2011119004 A
- WO 2016064087 A
- WO 2011119004 A2
- CN 103333935 A
- WO 2015032761 A

**Littérature non-brevet citée dans la description**

- **TAKESHITA et al.** Mass production of D-psicose from D-fructose by a continuous bioreactor system using immobilized D-tagatose 3-epimerase. *Journal of bioscience and bioengineering*, 2000, vol. 90, 453-455